# EUROPEAN PATENT SPECIFICATION

(11) **EP 1 514 844 B1**
(45) Date of publication and mention of the grant of the patent: **02.06.2010**
(21) Application number: 03727519.5
(22) Date of filing: 23.05.2003
(51) Int. Cl.: C01B 39/48, C01B 37/02, B01J 29/04, C10G 11/05, C07C 15/085, C07C 5/13, C07C 2/54

(54) **MICROPOROUS CRYSTALLINE ZEOLITE MATERIAL (ZEOLITE ITQ-22), SYNTHESIS METHOD THEREOF AND USE OF SAME AS A CATALYST**
MIKROPORÖSES KRISTALLINES ZEOLITHMATERIAL (ZEOLITH ITQ-22), SYNTHESEVERFAHREN DAFÜR UND VERWENDUNG DAVON ALS KATALYSATOR
MATIERE CRISTALLINE MICROPOREUSE DE NATURE ZEOLITIQUE (ZEOLITE ITQ-22) SON PROCEDE DE SYNTHESE ET SON UTILISATION EN TANT QUE CATALYSEUR

(30) Priority: 23.05.2002 ES 200201249; 14.02.2003 ES 200300444; 30.04.2003 ES 200301058
(43) Date of publication of application: 16.03.2005
(73) Proprietor: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, Avelino, Inst. de. T. Quimica, 46022 Valencia (ES); REY GARCIA, Fernando, Inst. de. T. Quimica, 46022 Valencia (ES); VALENCIA VALENCIA, Susana, Inst. de. T. Quimica, 46022 Valencia (ES); MARTINEZ TRIGUERO, Luis Joaquin, Inst. T. Quimica, 46022 Valencia (ES)
(74) Representative: Ungria Lopez, Javier
(86) International application number: PCT/ES2003/000246
(87) International publication number: WO 2003/099719

(56) References cited:
- WO-A-98/17581
- US-A- 5 068 096

## Description

### TECHNICAL FIELD OF THE INVENTION

This invention belongs to the technical field of microporous crystalline materials of zeolitic nature, useful as catalysts or components of catalysts for separation and transformation processes of organic compounds.

### STATE OF THE ART PRIOR TO THE INVENTION

Zeolites are microporous crystalline materials formed from a crystal lattice of TO₄ tetrahedra which share all their vertices giving rise to a three-dimensional structure containing channels and/or cavities of molecular dimensions. Their composition is variable and T in general represents atoms with formal oxidation state +3 or +4, such as for example Si, Ge, Ti, Al, B, Ga, and so on. When any of the T atoms has an oxidation state less than +4, the crystal lattice formed displays negative charges which are compensated by means of the presence of organic or inorganic cations in the channels or cavities. Organic molecules and H₂O can also be housed in those channels and cavities so, in a general manner, the chemical composition of the zeolites can be represented by means of the following empirical formula:

**x (M_{1/n}XO₂) : y YO₂ : z R : w H₂O**

wherein M is one or several organic or inorganic cations of charge +n; X is one or several trivalent elements; Y is one or several tetravalent elements, generally Si; and R is one or several organic substances. Although by means of post-synthesis treatment the nature of M, X, Y and R and the values of x, y, z and w can be varied, the chemical composition of a zeolite (as synthesised or following its calcination) possesses a range characteristic of each zeolite and of its method of preparation.

The crystalline structure of each zeolite, with a specific system of channels and cavities, gives rise to a characteristic X-ray diffraction pattern, which enables them to be differentiated from each other.

Many zeolites have been synthesised in the presence of an organic molecule which acts as a structure directing agent. The organic molecules which act as structure directing agents (SDA) generally contain nitrogen in their composition and can give rise to stable organic cations in the reaction medium.

The mobilisation of precursor species during the synthesis of zeolites can be carried out in the presence of OH⁻ groups and basic medium, which can be introduced as the hydroxide of the SDA itself, such as for example tetrapropylammonium hydroxide in the case of zeolite ZSM-5. Fluoride ions can also act as mobilising agents in the synthesis of zeolites, for example patent EP-A-337479 describes the use of HF in H₂O at low pH as a mobilising agent of silica for the synthesis of zeolite ZSM-5. Nevertheless, the use of fluoride ions in the synthesis is less desirable from the industrial point of view than the use of OH⁻, given that the presence of fluoride ions requires the use of special materials in the synthesis equipment as well as specific treatment of the waste waters and gases.

Fluid catalytic cracking (FCC), or its variant deep catalytic cracking (DCC), together with steam cracking, are the units which most contribute to the production of C3-C5 olefins. Moreover, FCC contributes approximately 30% of the gasoline stream in the refinery. The production of propylene in the FCC can be incremented by modifying the operating conditions of the unit, such as increasing the temperature of the reactor, for example. Nevertheless, this solution entails a considerable increase in gases and especially in undesired dry gas. Better results are obtained using new compositions of catalysts involving the use of zeolite mixtures. The use of zeolite ZSM-5 as additive in FCC catalysts also leads to an increase in C3 and C4 olefins (see for example patents US-3758403, US-3769202; US-3894931; US-3894933; US-3894934; US-3926782; US-4309280; US-4309279; and US-437458 and Buchanan, J.S. and Adewuyi, Y-G., Applied Catalysis: A General, 134, 247 (1996*)*; Madon, R.J., Journal of Catalysis 129 (1), 275 (1991*).* Nevertheless, it is known (Studies in Surface Science and Catalysis, vol. 76, 499 (1993*)*) that the introduction of zeolite ZSM-5 produces a decrease in dry gas, and an increase in the proportion of aromatics in the gasoline. This is particularly important from the point of view both of producing an increase in the yield of C3-C5 olefins, and especially propylene, and of obtaining a high yield of high octane/barrel gasoline with a low aromatics content, finding other alternative zeolites to zeolite ZSM-5 which, acting as an additive of zeolite faujasite Y, would provide light olefins with minimum loss of gasoline. Numerous zeolites have been studied in this direction such as zeolite MCM-22, Omega, L, mordenite BEA and ITQ-7 (see for example: J. Catal. 165, 102 (1997*);* Stud. Surf. Sci. and Catal. 46, 115 (1989*);* the patents: US-5314612; EP-489324; US-474292; US-4137152; EP-350331; FR-2661621; WO-0170905).

Nevertheless, the need persists to improve known procedures by means of using new catalysts with better performance in cracking.

With regard to the use of zeolites as catalysts in alkylation processes of aromatics, cumene is of particular interest as a raw material for the production of phenol and acetone. Numerous works have been developed using acid catalysts. A general reference on catalysts and processes used can be found in: "Encyclopedia of Chemical Processing and Design", J.J. McKezta and W.A. Cunningham Editors, V.14, pp. 33-55 (1982). The alkylation process of benzene with propylene, as well as seeking a high conversion of propylene and a high selectivity to the monoalkylated product isopropylbenzene (cumene), also requires minimising the quantity of n-propyl benzene (NPB) formed. Given that it is difficult to separate cumene and NPB by conventional methods, such as by distillation, it can be understood that the yield of NPB has to be as low as possible and in all cases very low, during the alkylation of benzene with propylene.

Zeolites have been used as catalysts for the alkylation of aromatics and so, for example, patent US-429457 describes zeolite ZSM-5 as a catalyst for alkylation of benzene with propylene.

Nevertheless, due probably to the small diameter of its channels, this zeolite displays low selectivity for the desired process. There also exist numerous patents which describe the use of Faujasite and modified Faujasites as catalysts for the production of cumene by alkylation of benzene with propylene. Zeolite Y can be used as a catalyst but it is necessary to work with high benzene/propylene ratios in the feed. This leads to high costs in the recycling of benzene. Zeolite Beta has also been claimed as a catalyst for alkylation of benzene with propylene in various patents such as for example: US-4891458, US-5030786, EP-432814, EP-439632; EP-629599. This zeolite produces good results in activity and selectivity, but its behaviour could be better both with regard to selectivity towards NPB and as far as the stability of the catalyst is concerned. The use of a new zeolite (MCM-22) has recently been claimed.

The object of the present invention is to provide a material of a nature that can advantageously be applied in processes of separation and transformation of organic compounds, and more particularly its use as an active zeolitic compound for the cracking of organic compounds and more specifically hydrocarbon fractions derived from natural or synthetic petroleums, as well as in the alkylation process of aromatics.

The pore topology of this new zeolite confers on it properties for acting as an additive of zeolite faujasite Y in catalysts for fluid catalytic cracking (FCC) units, providing high yields of light olefins with a high octane-barrel number of the gasoline produced, as well as reduction in the content of aromatics in the gasoline in comparison with the base catalyst.

### DESCRIPTION OF THE INVENTION

The present invention achieves the objectives defined above by means of a microporous crystalline material of zeolitic nature, also identified as "ITQ-22" or "zeolite ITQ-22", its method of preparation in the absence of fluoride ions and its applications. This material, in both its calcined form and synthesised uncalcined, has an X-ray diffraction pattern that is different from that of other known zeolitic materials and which is therefore characteristic of this material.

The X-ray diffraction pattern of the material ITQ-22, as synthesised, has been obtained by the powder method employing a fixed divergence slit and using K_{α} radiation from Cu. This pattern is characterised by the values of angle 2θ (degrees) and relative intensities (I/I₀) of the most intense reflections which are shown in Table I, with I₀ being the intensity of the most intense peak which is assigned a value of 100. The relative intensities have been expressed in the following terms: m = medium intensity (between 20 and 40%); s = strong intensity (between 40 and 60%) and vs = very strong intensity (between 60 and 100%).

**Table I**

| 2θ (degrees) ± 0.5 | Intensity (I/I₀) |
|---|---|
| 8.02 | vs |
| 8.43 | vs |
| 9.31 | m |
| 10.64 | vs |
| 20.26 | m |
| 21.81 | s |
| 22.53 | vs |

Once calcined and in the anhydrous state, the material accords with the general formula

***x* (H XO₂) : *y* YO₂ : *z* GeO₂ : (1 - z) SiO₂**

wherein "*x*" possesses a value less than 0.2, preferably less than 0.1 and can take the value zero; "*y"* has a value less than 0.1, preferably less than 0.05 and can take the value zero; "*z*" possesses a value less than 0.8, preferably between 0.005 and 0.5 and can take the value zero; X is one or several chemical elements of oxidation state +3 (Al, Ga, B, Fe, Cr) and Y is one or several chemical elements with oxidation state +4 other than Si and Ge (Ti, Sn, V).

Nevertheless, depending on the synthesis method and on the calcination or later treatments, it is possible for defects to exist in the crystal lattice, which are manifested by the presence of Si-OH (silanol) groups. These defects have not been included in the above empirical formula.

Table II shows the values of angle 2θ (degrees) and relative intensities (I/I₀) of the most intense reflections of the powder X-ray diffractogram zeolite ITQ-22 after being calcined in order to eliminate organic compounds occluded in its interior, where m, s, and vs have the same meanings as in Table I.

**Table II**

| 2θ (degrees) ± 0.5 | Intensity (I/I₀) |
|---|---|
| 6.97 | m |
| 8.00 | vs |
| 8.40 | vs |
| 9.28 | m |
| 10.62 | vs |
| 20.18 | m |
| 21.74 | m |
| 22.54 | vs |

The positions, widths and relative intensities of the peaks depends to a certain degree on the chemical composition of the material, as well as on the degree of hydration and crystal size. In particular, when the lattice is composed solely of silicon and germanium oxides, with a ratio Si/Ge = 3.8 and it has been synthesised using the quaternary ammonium cation 1,5-bis (methylpyrrolidinium) pentane as structure directing agent, the material as synthesised displays an X-ray diffraction pattern like that shown in Figure I. This diagram is characterised by the values of angle 2θ (degrees) and relative intensities (I/I₀) that are shown in Table III. The relative intensities have been expressed in the following terms: w = weak intensity (between 0 and 20%); m = medium intensity (between 20 and 40%); s = strong intensity (between 40 and 60%) and vs = very strong intensity (between 60 and 100%).

**Table III**

| 2θ (degrees) ± 0.5 | Intensity (I/I₀) |
|---|---|
| 6.96 | w |
| 7.12 | w |
| 8.02 | vs |
| 8.43 | vs |
| 9.31 | m |
| 10.64 | vs |
| 10.95 | w |
| 13.83 | w |
| 16.11 | w |
| 16.31 | w |
| 18.68 | w |
| 19.58 | w |
| 19.95 | w |
| 20.11 | m |
| 20.26 | m |
| 20.68 | w |
| 21.54 | w |
| 21.81 | s |
| 22.27 | w |
| 22.53 | vs |
| 22.70 | m |
| 22.91 | w |
| 23.03 | w |
| 23.18 | w |
| 24.22 | w |
| 24.33 | w |
| 25.30 | w |
| 26.44 | w |
| 27.65 | w |
| 28.14 | w |
| 28.52 | w |
| 29.03 | w |
| 29.69 | w |
| 29.90 | w |
| 32.28 | w |
| 33.26 | w |
| 35.98 | w |

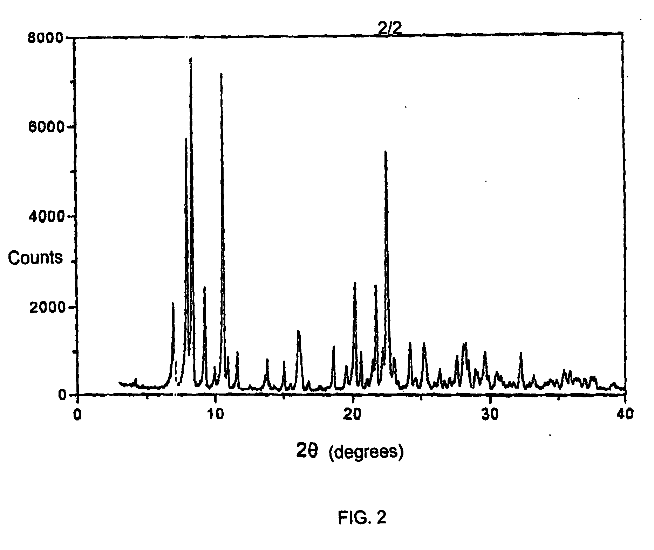

The X-ray diffraction pattern of the above sample of ITQ-22, after being calcined at 580°C in order to eliminate organic compounds occluded in its interior, is shown in Figure 2. This diffractogram is characterised by the values of angle 2θ (degrees) and relative intensities (I/I₀) that are shown in Table IV, where w, m, s and vs have the same meanings as in Table III. The comparison of the X-ray diffractograms corresponding to zeolite ITQ-22 as synthesised and in the calcined state demonstrate the thermal stability of the material.

**Table IV**

| 2θ (degrees) ± 0.5 | Intensity (I/I₀) |
|---|---|
| 6.97 | m |
| 7.12 | w |
| 8.00 | vs |
| 8.40 | vs |
| 9.28 | m |
| 9.97 | w |
| 10.62 | vs |
| 10.92 | w |
| 11.62 | w |
| 13.81 | w |
| 15.04 | w |
| 16.04 | w |
| 16.14 | w |
| 16.26 | w |
| 18.63 | w |
| 19.52 | w |
| 20.18 | m |
| 20.63 | w |
| 21.48 | w |
| 21.74 | m |
| 22.21 | w |
| 22.54 | vs |
| 23.04 | w |
| 24.17 | w |
| 25.23 | w |
| 26.33 | w |
| 27.58 | w |
| 28.02 | w |
| 28.22 | w |
| 28.42 | w |
| 28.92 | w |
| 29.62 | w |
| 32.24 | w |

The present invention also refers to the preparation method of ITQ-22. This is carried out in basic medium, in the absence of fluoride ions and comprises heat treatment at a temperature between 80 and 200°C, preferably between 130 and 200°C, of a reaction mixture containing a source of SiO₂ (such as for example tetraethylorthosilicate, colloidal silica, amorphous silica), optionally a source of GeO₂, 1,5-bis (methylpyrrolidinium) pentane, and water. The organic cation 1,5-bis(methylpyrrolidinium) pentane is added to the reaction mixture in the form of salt (for example, a halide, preferably, chloride or bromide) or hydroxide.

Optionally, it is possible to add a source of tetravalent element or elements Y other than Si and Ge, preferably Ti, V, Sn and/or trivalent elements X, preferably Al, B, Ga, Fe, Cr The addition of this element or elements can be done prior to heating of the reaction mixture or in an intermediate moment during that heating. It can occasionally be convenient to also add crystals of ITQ-22 (between 0.01 and 20% by weight with respect to the inorganic oxides taken as a whole, preferably between 0.05 and 10% by weight) as promoters of the crystallisation (sowing) at some stage of the preparation. The composition of the reaction mixture accords with the general empirical formula:

***r* ROH : *t* X₂O₃ : *u* YO₂ : *v* GeO₂ : SiO₂ : *w* H₂O**

wherein X is one or several trivalent elements, preferably Al, B, Ga, Fe, Cr; Y is one or several tetravalent elements other than Si and Ge, preferably Ti, Sn, V; R is 1,5-bis (methylpyrrolidinium) pentane; and the values of *r, t, u, v* and *w* vary in the ranges
*r* = ROH/SiO₂ = 0.01-1.0, preferably 0.1-1.0
*t* = X₂O₃/SiO₂ = 0-0.1, preferably 0-0.05
*u* = YO₂/SiO₂ = 0-0.1, preferably 0-0.05
*v* = GeO₂/SiO₂ = 0-4, preferably 0.005-1
*w* = H₂O/SiO₂ = 1-50

The heat treatment of the reaction mixture can be done statically or with stirring of the mixture. Once the crystallisation is complete, the solid product is separated by filtration or centrifugation and dried. The later calcination at temperatures above 350°C, preferably between 400 and 900°C, produces the decomposition of organic remains occluded in the interior of the zeolite and their exit, leaving the zeolitic channels free. The calcination can be done in the presence of N₂ followed by calcination in air, or direct calcination in air can be carried out, or an extraction of the organic matter with mineral or organic acids can be performed, or the organic material can be eliminated by treatment with ozone.

After having been calcined, the material is pelletised. The means by which the catalyst is pelletised are well known in the literature, using a diluent such as for example SiO₂ or Al₂O₃ or a clay, zirconium, magnesium oxide or mixture thereof, in proportions of zeolite/diluent of between 20 and 95%, preferably between 40 and 90% by weight.
In an alternative embodiment, the calcined catalyst can be treated with an aqueous solution of a mineral acid such as for example HNO₃, H₂SO₄, H₃PO₄, HClO₄.

By means of treatment of that aqueous solution of mineral acid, a pH of between 0 and 1.5 is reached, at temperatures between 20 and 100°C in a time period between 10 and 400 minutes, depending on the concentrations of acid and the treatment temperature. The ratio of catalyst to aqueous solution of acid lies In an alternative embodiment, the calcined catalyst can be treated with an aqueous solution of a mineral acid such as for example HNO₃, H₂SO₄, H₃PO₄, HClO₄.

By means of treatment of that aqueous solution of mineral acid, a pH of between 0 and 1.5 is reached, at temperatures between 20 and 100°C in a time period between 10 and 400 minutes, depending on the concentrations of acid and the treatment temperature. The ratio of catalyst to aqueous solution of acid lies between 0.05 and 1, and preferably between 0.1 and 0.5 by weight.

The resulting material, whether or not treated with acid, but containing acid centres in its structure, once it has been calcined at a temperature between 450 and 700°C, is used as a catalyst.

The ITQ-22 material can be used in the following applications:
As an additive of catalytic cracking catalysts for hydrocarbons, and for organic compounds in general.
As a component of hydrocracking and gentle hydrocracking catalysts.
As a component or additive of isomerisation catalysts for light paraffins.
As a component of deparaffining and isodeparaffining catalysts.
As an alkylation catalyst of isoparaffins with olefins and alkylation of aromatics and aromatics substituted with olefins, alcohols or polyalkylated aromatics, and more specifically as a catalyst for the alkylation of benzene with propylene.
As a catalyst in acylation reactions, preferably in acylation reactions of substituted aromatic compounds using acids, acid chlorides or anhydrides of organic acids as acylating agents.
As catalysts in oxidation reactions, preferably of the Meerwein-Pondorf-Verley type.
An especially preferred use is as an additive of catalytic cracking catalysts for hydrocarbons, and for organic compounds in general. Zeolite ITQ-22 has a three-dimensional topology with a system of pores and dimensions different from any other zeolite used so far in FCC catalysts. This zeolite contains pores formed by of 8, 10 and 12 membered rings connected, and it is precisely this topology which gives rise to a specific behaviour in cracking catalysts.

In its use as a cracking catalyst, the zeolitic material ITQ-22 can be the only zeolitic component of the catalyst embedded in a matrix, or it can be accompanied by at least one second zeolitic component, both embedded in a matrix.

When ITQ-22 is accompanied by another zeolitic component or components, said second zeolitic component can be formed of zeolitic structures selected among zeolitic structures containing pores delimited by rings of 14 members, such as for example SSZ-24, CIT-5, UTD-1; zeolitic structures containing pores delimited by rings of 12 members, such as for example zeolite Beta, ITQ-7, zeolite faujasite Y, SSZ-33; zeolitic structures containing pores delimited by rings of 11 members, such as for example NU-86; zeolitic structures containing pores delimited by rings of 10 members, such as for example ITQ-13, ZSM-5, SAPO-11, MCM-22, and mixtures thereof.

Moreover, for the cracking process the catalyst can consist partially or wholly of a single type of particle with the material ITQ-22 and the said at least second component being present in the catalyst in the same particle, embedded in a matrix. Said particle preferably comprises at least two zeolitic components which are ITQ-22 and zeolite faujasite Y in one or more of its variants selected from the group formed from zeolite faujasite Y, ultrastable zeolite faujasite Y, zeolite faujasite Y fully exchanged with rare earths, zeolite faujasite Y partially exchanged with rare earths, ultrastable zeolite faujasite Y fully exchanged with rare earths, ultrastable zeolite faujasite Y partially exchanged with rare earths and mixtures thereof. Preferably, said second zeolitic component of the type faujasite Y is present in quantities between 0.1 and 99.9% by weight with respect to the total weight of zeolitic components, with the weight of zeolite ITQ-22 being between 0.1 and 60% by weight with respect to the total weight of zeolitic components. The rest of the catalyst composition is formed by the matrix containing components well known in the art, such as kaolin, alumina and silica, being able to also contain P₂O₅.

When zeolite ITQ-22 is used in cracking together with at least one other zeolitic component and wherein the zeolitic components are embedded in the same particle, at least some of the particles of the catalytic cracking catalyst preferably comprise:
- 0.1-40% by weight of the zeolitic material ITQ-22 with respect to the total weight of zeolitic components
- 0.1-99.9% by weight of the zeolite faujasite Y with respect to the total weight of zeolitic components.

In its use in cracking processes, zeolite ITQ-22 can form part of a catalyst partially or wholly constructed of at least two types of particle in which the zeolitic material ITQ-22 and said at least one second zeolitic material are embedded in a matrix, the zeolitic material ITQ-22 and said second zeolitic component being present in different particles, the catalyst being a physical mixture of particles of different nature. So, in this case, each zeolite would be incorporated separately into a matrix. The final mixture of the catalyst would be formed by a mixture of at least two types of particle, with each type of particle incorporating a different zeolitic component. The final mixture would preferably be formed by a type of particle that includes ITQ-22 in a matrix, and by a second type of particle that includes zeolite faujasite Y in any of the different forms of faujasite Y, embedded in a second matrix. An FCC catalyst would be formed by this type of said mixture of particles; particles with ITQ-22 and particles with zeolite faujasite Y in any of its two forms.

When zeolite ITQ-22 is used in a cracking process with another zeolitic component or components, with the zeolitic components being embedded in different types of particle, the catalytic cracking catalyst preferably comprises:
- 0.1-40% by weight of particles containing zeolite ITQ-22 with respect to the sum of zeolitic components, in which each particle comprises 10-70% by weight of zeolite ITQ-22;
- 0.1-99.9% by weight of particles containing a conventional catalytic cracking catalyst based on faujasite type zeolite, in which the percentages are indicated on the sum of zeolitic components.

In the case that the catalyst for use in cracking comprises ITQ-22 and another zeolitic component or components, contained in particles of different type, said catalyst can have a composition in which there exists at least a third type of particle, in which a third zeolite would be included in a matrix. This third zeolite is preferably ZSM-5.

Of course, and as is known in the art, a final catalyst could also contain other particles with the aim of, for example, and without being limiting, improving fluidisation, trapping contaminants (such as for example metals, nitrogen, Na⁺), converting beds, trapping SOx. In particular, an FCC catalyst could contain these types of additional particles mentioned.

In the catalytic cracking catalyst described which contains ITQ-22, zeolite faujasite Y and ZSM-5, a physical mixture of separate particles is preferred in which the zeolitic components are to be found in the following proportions: Zeolite faujasite Y at no less than 20% by weight with respect to the total weight of zeolitic components; zeolite ITQ-22 + ZSM-5 at a maximum of 80% by weight, the ratio by weight between zeolite ZSM-5 and zeolite ITQ-22 lying between 10 and 0.

When the catalyst contains zeolite ITQ-22 and at least two other zeolitic components, contained in particles of different type, the third component is preferably zeolite ZSM-5.

For its use in cracking, the catalyst can comprise more than three types of different particles, in particular FCC catalysts which can also contain other particles for improving fluidisation, etc.

The catalyst particles include components that are not active in the catalytic process though they are necessary in order to conform them, such as binders and/or conventional additives such as clays, among others.

The zeolitic components can include one or more T^{IV} elements, such as for example Si and also other T^{III} element or elements, preferably selected from the group made up of Al, Fe, Ge, Ga, Zn, Ti, B and mixtures thereof. It can also include phosphorus. Likewise, they can be exchanged with divalent and/or trivalent ions or with rare earths. Also optionally, they can contain compounds of vanadium, and optionally cerium, added in a stage subsequent to the synthesis of the zeolite.
Both when the zeolitic components are to be found in the same particle and when the zeolitic components are to be found in separate particles, zeolite faujasite Y can be added to the cracking catalyst in various forms, such as zeolite HY, ultrastable zeolite faujasite Y (USY), zeolite faujasite Y totally exchanged with rare earths (REY) or with rare earths and H⁺ (HREY), zeolite USY partially exchanged with rare earths (REUSY), or zeolite type Y exchanged with rare earths and calcined (CREY). When a catalytic cracking catalyst comprises ITQ-22 with at least two other zeolitic components in separate particles, each type of catalyst particle can comprise one or more or the zeolitic components.
Both in the case of the catalyst containing ITQ-22 as the sole zeolitic component and in the case of its containing another zeolitic component or components, in order to form the catalyst particles a matrix is used containing at least one binder such as silica, alumina, silica-alumina, P₂O₅ and mixtures thereof. The final particles of catalyst can also contain conventional additives for catalytic cracking such as clays.

In particular, in FCC catalysts, the catalyst can contain any conventional material, such as for example kaolin, in order to conform catalysts with various sizes of particle. When an FCC catalyst is prepared, a suspension can be made and atomised in order to form the particles of the catalyst. In the case of FCC units, particles of between 60 and 200 micrometres are preferred.

The different zeolitic components of the zeolitic material ITQ-22 which form part of a catalyst for use in cracking can include one or more T^{IV} elements, such as for example Si and Ge and also include at least one T^{III} element. Said T^{III} element is preferably selected from the group made up of Al, Fe, Ge, Ga, Zn, Ti, B and mixtures thereof.

In the case of catalytic cracking in FCC units, zeolite ITQ-22 can be modified by means of the addition of phosphorus. In its initial composition, the molar ratio between T^{IV} and T^{III} lies between 8 and 10000 and preferably between 20 and 1000. The cracking catalyst can also contain one or more alkaline metals. In no case may the quantity of alkaline metal, if there is any, exceed 0.25% by weight of Na₂O.

The zeolitic components that can form part of the cracking catalyst, including the zeolitic material ITQ-22, as has been stated earlier, can contain phosphorus. The quantity of phosphorus present preferably lies between 0 and 8% by weight with respect to zeolite ITQ-22. The phosphorus can be incorporated by impregnation in a, for example aqueous, solution of at least one acid or salt chosen from the group consisting of H₃PO₄, (NH₄)₃PO₄, (NH₄)₂HPO₄, (NH₄)H₂PO₄. The product obtained is calcined at a temperature between 350 and 700 °C.

The zeolitic components that can form part of the cracking catalyst, including the zeolitic material ITQ-22, can be exchanged with divalent and/or trivalent ions or they can be exchanged wholly or partially with rare earths.
For use in cracking, the catalyst can contain compounds of vanadium and optionally cerium added in a stage subsequent to the synthesis of zeolite ITQ-22.

For use in cracking the catalyst particles can have a size between 20 and 400 micrometres. In a preferred embodiment the catalyst is formed from particles with a size between 60 and 200 micrometres.

The zeolite ITQ-22 can be used in an FCC cracking process and in deep catalytic cracking (DCC), a process which consists of placing the feed in contact with the catalyst in a reactor for a length of time between 0.1 and 80 s and recovering the resulting product.

By means of using zeolite ITQ-22, gasoline is obtained with a lower content of aromatics, with a higher content of olefins and isoparaffins, less dry gas, higher octane-barrel number and a greater propylene/propane ratio than when a catalyst that does not contain ITQ-22 is used.

An especially preferred additional use for zeolite ITQ-22 is as a catalyst in an alkylation reaction. In said alkylation reaction, a suitable quantity of the catalyst is used along with an alkylating agent selected among olefins, alcohols, polyalkylated aromatic compounds and mixtures thereof in the alkylation of aromatic compounds.

Said alkylation reaction is preferably conducted with a molar ratio of alkylating agent to starting aromatic compound of between 2 and 20, in the presence of the catalyst.

When the alkylating agent is selected among an olefin, alcohol and mixtures thereof, said olefin and said alcohol preferably contain from between 2 to 20 carbon atoms.

In a preferred manner, the starting aromatic compound is selected among a group formed of benzene, naphthalene, anthracene, phenanthrene and substituted derivatives thereof, and still more preferably, the starting aromatic compound is benzene.
The starting aromatic compound can also be selected among alkylbenezene, alkylanthracene, alkylphenanthrene, hydroxybenzene, hydroxynaphthalene, hydroxyanthracene, hydroxyphenanthrene, alcoxybenzene, alcoxynaphthalene, alcoxyanthracene and alcoxyphenanthrene.

The alkylating agent is preferably a polyalkylated aromatic compound and the starting aromatic compound is a non-alkylated aromatic compound, so that during the alkylation at least one alkyl group is transferred from the polyalkylated aromatic compound to the starting aromatic compound.

When the alkylating agent is a polyalkylated aromatic compound, the alkyl group or the alkyl groups of said polylalkylating agent can contain a variable number of carbon atoms. In a preferred way, said alkyl group or groups can contain from 2 up to 20 carbon atoms, preferably from 6 to 20 carbons atoms.

When the alkylating agent is a polyalkylated aromatic compound and the starting aromatic compound is a non-alkylated aromatic compound, said starting aromatic compound is preferably selected among benzene, naphthalene, anthracene, phenanthrene, substituted benzene, substituted naphthalene, substituted anthracene and substituted phenanthrene.

The alkylating agent is preferably a polyalkylated aromatic agent and the starting aromatic compound is benzene. Still more preferably, said polyalkylated aromatic compound is polyisopropylbenzene and the starting aromatic compound is benzene, so that the alkylation of benzene with propylene produces cumene.
A preferred use of ITQ-22 therefore refers to the alkylation of benzene with an alkylating agent selected among propylene, ethylene, ethyl alcohol, propyl alcohol and mixtures thereof.

According to the use of zeolite ITQ-22 in alkylation, the alkylation reaction is conducted at a reaction temperature of between 60 and 350°C, and preferably between 80 and 300°C.

The alkylation reaction is conducted at sufficient pressure for maintaining a liquid phase, at least partially, preferably in a range between 1.4 and 7.0 MPa, and more preferably between 1.4 and 4.1 MPa.

In the alkylation reaction, the spatial velocity (WHSV) of reagents lies between 0.2 and 150 hours⁻¹ and preferably between 0.5 and 10 hours⁻¹.

The alkylation reaction of benzene with propylene is preferably conducted under the following conditions:
reaction temperature lying between 60 and 350°C, and
preferably between 80 and 300°C; the pressure at which it is carried out is also sufficient for maintaining a liquid phase, at least partially, preferably being in a range between 1.4 and 7.0 MPa, and more preferably between 1.4 and 4.1 MPa; the spatial velocity (WHSV) of reagents lies between 0.2 and 150 hours⁻¹ and preferably between 0.5 and 10 hours⁻¹ and the benzene/propylene molar ratio is between 2 and 20, and preferably 2 and 15.

When ITQ-22 is applied in its acid form in the alkylation of aromatics with olefins or alcohols, and more specifically when it is used as a catalyst in the alkylation of benzene with propylene it turns out to be a highly active catalyst, and with a surprisingly low selectivity for the production of NPB. Moreover, the selectivity to cumene can be increased by introducing suitable quantities of alkaline or alkaline earth metals or metallic cations by means of ion exchange in the material. Its selectivity can also be increased by eliminating surface acidity by means of extraction of trivalent cations from the lattice, such as for example Al and/or B, by means of treatment with mineral acids or other chemical agents capable of extracting those elements. The cationic exchange treatments or leaching stated above permit a reduction in the formation of polyalkylated products.
When the ITQ-22 material contains Ti, it is especially useful as a catalyst in epoxidation reactions of olefins, oxidation of alkanes, oxidation of alcohols and oxidation of thioethers to sulphoxides and sulphones using organic or inorganic hydroperoxides, such as for example H₂O₂, tertbutylhydroperoxide, cumene hydroperoxide, as oxidising agents, in the amoximation of ketones, and more specifically of cyclohexanone to cyclohexanone oxime with NH₃ and H₂O₂. When the ITQ-22 material contains Sn it is particularly useful as a catalyst in Baeyer-Villiger oxidation reactions in which H₂O₂ is used as an oxidising agent.

### BRIEF DESCRIPTION OF THE FIGURES

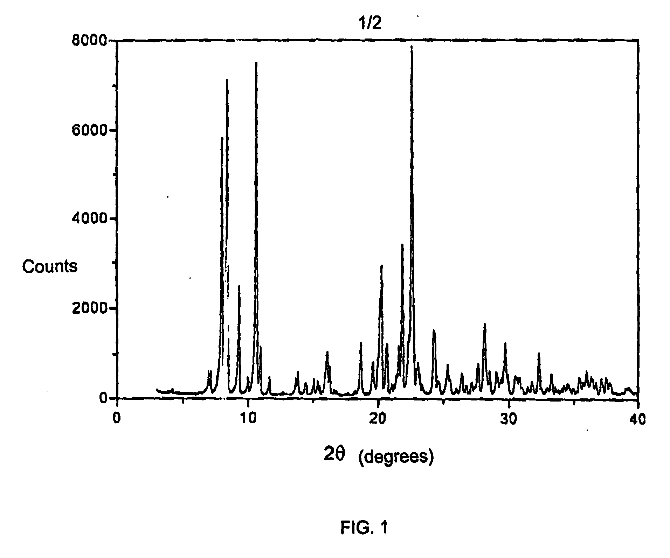

Figure 1 represents the X-ray diffraction pattern of a sample of ITQ-22 as synthesised, in which the lattice consists solely of silicon and germanium oxides, with a ratio Si/Ge = 3.8 and which has been synthesised using the quaternary ammonium cation 1,5-bis (methylpyrrolidinium) pentane as structure directing agent.
Figure 2 shows the X-ray diffraction pattern of the same sample of ITQ-22 after being calcined at 580°C in order to eliminate organic compounds occluded in its interior.

### EXAMPLES

### EXAMPLE 1:

This example illustrates the preparation of the dihydroxide of 1,5-bis (methylpyrrolidinium) pentane. In a 500 ml flask, 18.7 g of 1,5-dibromopentane, 20 g of 1-methylpyrrolidine and 300 ml of acetone are mixed. The mixture is left to reflux for 24 hours, after which the precipitated solid is separated by decantation and then washed several times with acetone. Finally it is vacuum dried and 31.9 g of a white solid are obtained. The analysis of elements and the nuclear magnetic resonance spectrum in D₂O of this solid indicate that it is the desired product, namely , 1,5-bis (methylpyrrolidinium) pentane dibromide.
The dihydroxide form of the structure directing agent is obtained by means of anion exchange using a Dowex SBR resin (in molar excess). The exchange is carried out with stirring of the fluid bed of resin in hydroxide form and an aqueous solution of the bromide of the cation for one night. The solution obtained is titrated with HCl (aq.) using phenolphthalein as indicator, with an exchange efficiency of 95% being obtained. This solution can be concentrated in the rotary evaporator for use in the synthesis of molecular sieves, for example at a concentration of 0.5-1 N.

### EXAMPLE 2:

This example illustrates the preparation of ITQ-22 by means of using the cation 1,5-bis (methylpyrrolidinium) pentane.

6.17 g of tetraethylorthosilicate (TEOS) are added to 23.14 g of a solution of 1,5-bis (methylpyrrolidinium) pentane dihydroxide (R(OH)₂) containing 0.96 equivalents of hydroxide in 1000 g. In this mixture 1.55 g of germanium oxide are added, and it is left to evaporate with stirring until complete elimination of the ethanol deriving from the hydrolysis of the TEOS plus the necessary quantity of water so that the final composition of the gel is:

0.67 SiO₂ : 0.33 GeO₂ : 0.25 R(OH)₂ : 3.5 H₂O

The mixture obtained is introduced into an autoclave internally lined with polytetrafluoroethylene and is heated at 175°C for 14 days. The autoclave is then cooled, the contents are filtered, the solid is washed with water and dried at 100°C. The X-ray diffraction pattern is shown in Figure I and the list of the most characteristic peaks appears in Table III. Calcination at 580°C in air for 3 days permits occluded organic species to be eliminated. The X-ray diffraction pattern of the calcined zeolite ITQ-22 is shown in Figure 2 and the list of most characteristic peaks appears in Table IV and indicates that the material is stable during this process.

### EXAMPLE 3:

The following example illustrates the preparation of ITQ-22 of different composition.

12 g of tetraethylorthosilicate (TEOS) are added to 27.3 g of a solution of 1,5-bis (methylpyrrolidinium) pentane dihydroxide (R(OH)₂) containing 1.16 equivalents of hydroxide in 1000 g. In this mixture 0.6 g of germanium oxide are dissolved, and it is left to evaporate with stirring until complete elimination of the ethanol formed plus the excess of water so that the final composition is as follows:

0.91 SiO₂ : 0.09 GeO₂ : 0.25 R(OH)₂ : 3.5 H₂O

The mixture obtained is heated in autoclaves internally lined with polytetrafluoroethylene at 175°C for 14 days. The solid obtained after filtering, washing with distilled water and drying at 100°C is ITQ-22.

### EXAMPLE 4:

This example illustrates synthesis of Al-ITQ-22.

0.097 g of Al isopropoxide are added to 9 g of tetraethylorthosilicate (TEOS). After that, 20.68 g of a solution of 1,5-bis (methylpyrrolidinium) pentane dihydroxide (R(OH)₂) containing 1.16 equivalents of hydroxide in 1000 g are added, and then 0.45 g of germanium oxide are dissolved. The mixture is left to evaporate with stirring until complete elimination of the ethanol deriving from the hydrolysis of the TEOS plus the necessary quantity of water in order to achieve the stated final composition. The composition of the gel is: 0.91 SiO₂ : 0.09 GeO₂ : 0.005 Al₂O₃ : 0.25 R(OH)₂ : 3.5 H₂O

The mixture obtained is introduced into an autoclave internally lined with polytetrafluoroethylene and is heated to 175°C for 12 days.
The X-ray diffraction pattern of the solid obtained after filtering, washing with distilled water and drying at 100°C indicates that it is ITQ-22.

### EXAMPLE 5:

This example illustrates the preparation of a sample of Al-ITQ-22 with a chemical composition different from that of the above example.

0.325 g of Al isopropoxide are added to 11.05 g of tetraethylorthosilicate (TEOS). After that, 34.2 g of a solution of 1,5-bis (methylpyrrolidinium) pentane dihydroxide (R(OH)₂) containing 1.2 equivalents of hydroxide in 1000 g are added, and then 2.77 g of germanium oxide are dissolved. The mixture is left to evaporate with stirring until complete elimination of the ethanol deriving from the hydrolysis of the TEOS plus the necessary quantity of water in order to achieve the stated final composition. The composition of the gel is: 0.67 SiO₂ : 0.33 GeO₂ : 0.01 Al₂O₃ : 0.25 R(OH)₂ : 15 H₂O

The mixture obtained is introduced into autoclaves internally lined with polytetrafluoroethylene and is heated to 175°C for 12 days.
The X-ray diffraction pattern of the solid obtained after filtering, washing with distilled water and drying at 100°C indicates that it is ITQ-22. The solid obtained is calcined at 580°C for 3 hours and the X-ray diffraction pattern shows that the structure is maintained. The chemical analysis of the calcined zeolite indicates that it has a composition that accords with the molar ratios Si/Ge = 5 and (Si + Ge)/Al = 30.

### EXAMPLE 6:

This example illustrates the synthesis of Ti-ITQ-22.

0.3 g of Ti(IV) tetraethoxide are added to 9.26 g of tetraethylorthosilicate (TEOS). After that, 39.97 g of a solution of 1,5-bis (methylpyrrolidinium) pentane dihydroxide (R(OH)₂) containing 0.85 equivalents of hydroxide in 1000 g are added, and then 2.32 g of germanium oxide are dissolved. The mixture is left to evaporate with stirring until complete elimination of the ethanol deriving from the hydrolysis of the TEOS plus the necessary quantity of water in order to achieve the stated final composition. The composition of the gel is: 0.67 SiO₂ : 0.33 GeO₂ : 0.02 TiO₂ : 0.25 R(OH)₂ : 3.5 H₂O

The mixture obtained is introduced into an autoclave internally lined with polytetrafluoroethylene and is heated to 175°C for 16 days.
The X-ray diffraction pattern of the solid obtained after filtering, washing with distilled water and drying at 100°C indicates that it is ITQ-22.

### EXAMPLE 7:

This example illustrates the synthesis of Sn-ITQ-22.

To 5.00 g of tetraethylorthosilicate (TEOS), 15.12 g of a solution of 1,5-bis (methylpyrrolidinium) pentane dihydroxide (R(OH)₂) containing 1.20 equivalents of hydroxide in 1000 g are added, and then 1.26 g of germanium oxide are dissolved. After that a solution of 0.105 g of tin (IV) tetrachloride pentahydrate in 2 g of water is added. The mixture is left to evaporate with stirring until complete elimination of the ethanol deriving from the hydrolysis of the TEOS plus the necessary quantity of water in order to achieve the stated final composition. The composition of the gel is: 0.67 SiO₂ : 0.33 GeO₂ : 0.0083 SnO₂ : 0.25 R(OH)₂ : 3.5 H₂O

The mixture obtained is introduced into an autoclave internally lined with polytetrafluoroethylene and is heated to 175°C for 23 days.
The X-ray diffraction pattern of the solid obtained after filtering, washing with distilled water and drying at 100°C indicates that it is ITQ-22.

### EXAMPLE 8:

**This example illustrates the preparation of a component of the catalytic cracking catalyst containing zeolite type ITQ-22 previously activated in order to obtain the acid form at 580°C in air.**

With the aim of studying the activity and selectivity of zeolite ITQ-22 in the catalytic cracking of industrial fractions of petroleum, a catalyst was prepared mixing ITQ-22 type zeolite, in its acid form, as was described in example 5 and a silica (BASF D1111), both in powder form and in a composition of 23% and 77% by weight of ITQ-22 type zeolite, and SiO₂, respectively. The mixture, thoroughly homogenised, was made into cake form, crushed in a mortar and sieved taking the fraction between 0.59 and 0.84 mm in diameter.

### EXAMPLE 9:

**This example illustrates the use of zeolite ITQ-22 as additive of the catalytic cracking catalyst of a vacuum gasoil.**

The catalytic components described in example 8 were used as additive of a zeolite USY catalyst in the catalytic cracking of a vacuum gasoil whose characteristics are given in table V The catalysts are provided on two separate beds in the reactor as described in Journal of Catalysis 1997, 165 (1), p. 102*.* In the upper zone, 1.5 grams of zeolite USY catalyst are placed, and in the lower zone 1.34 g of catalyst of example 8.

**Table V. Properties of the vacuum gasoil**

| | |
|---|---|
| Density (1°5°C) g cc⁻¹ | 0.9172 |
| Aniline point (°C) | 79.2 |
| Sulphur (% by weight) | 1.65 |
| Nitrogen (ppm) | 1261 |
| Na (ppm) | 0.18 |
| Cu (ppm) | < 0.1 |
| Fe (ppm) | 0.30 |
| Ni (ppm) | 0.2 |
| V (ppm) | 0.40 |
| **ASTM D-1160 (°C)** | |
| 5% | 319 |
| 10% | 352 |
| 30% | 414 |
| 50% | 436 |
| 70% | 459 |
| 90% | 512 |
| VABP (°C) ¹⁾ | 435 |
| K (UOP) | 11.82 |
| Average molecular weight | 407 |
| Aromatic carbon (% by weight) | 22.96 |
| Naphthenic carbon (% by weight) | 15.16 |
| Paraffinic carbon (% by weight) | 61.88 |

| | |
|---|---|
| ¹⁾ VABP = volume average boiling point | |

The reaction conditions were 520°C, reaction time of 30 seconds, 2.34 grams of catalyst and catalyst/feed

| | |
|---|---|
| 70% | 459 |
| 90% | 512 |
| VABP (°C) ¹⁾ | 435 |
| K (UOP) | 11.82 |
| Average molecular weight | 407 |
| Aromatic carbon (% by weight) | 22.96 |
| Naphthenic carbon (% by weight) | 15.16 |
| Paraffinic carbon (% by weight) | 61.88 |

| | |
|---|---|
| ¹⁾ VABP = volume average boiling point | |

The reaction conditions were 520°C, reaction time of 30 seconds, 2.34 grams of catalyst and catalyst/feed ratios by weight of 0.59, 0.73, 0.95, 1.35 and 2.38, the quantities of gasoil fed being 3.76, 3.07, 2.35, 1.65 and 0.94 grams. The gases produced were analysed by gas chromatography, the liquids by simulated distillation (ASTM D-2887) and the coke was measured by analysis (IR) of the CO₂ formed during the combustion. The conversion is defined as the sum of the yields of gases H₂ and C₁ - C₄ coke and gasoline (boiling point < 235.4 °C).
The composition (PIONA: Paraffins, Isoparaffins, Olefins and Aromatics) and quality (RON: Research Octane Number) of the gasoline fraction were determined by gas chromatography using the "detailed hydrocarbons analysis" computer program supplied by VARIAN and the correlations shown in the publication: Lugo, H.J., Ragone, G., and Zambrano, J., Ind. Eng. Chem. Res. 38, 2171 (1999*).* The results obtained by interpolation at 80% of total conversion are shown in table VI. The use of the zeolite ITQ-22 as additive of zeolite USY (USY/ITQ-22 Catalyst) increases the yield of olefins C3 and C4 with respect to the base catalyst (USY), with a slight drop in the yield of gasoline. Also, the gasoline obtained has a lower aromatics content when the catalyst contains the additive ITQ-22.

It is important to note that the propylene/propane ratio obtained is greater when ITQ-22 is used as additive, this result being highly beneficial from the point of view of a possible industrial process.

**Table VI. Selectivities and ratios of interest in the catalytic cracking of gasoil at 520°C and feed time of 30 s. Fresh additive.**

| CATALYST | USY | USY/ITQ-22 |
|---|---|---|
| CAT/OIL (g/g) | 1.50 | 1.37 |
| CONVERSION % | 80 | 80 |
| | | |
| GASOLINE % | 45.9 | 40.3 |
| GASES % | 28.1 | 33.0 |
| COKE % | 6.0 | 6.7 |
| C1-C4 fraction | | |
| HYDROGEN % | 0.12 | 0.10 |
| METHANE % | 0.88 | 0.88 |
| ETHANE % | 1.04 | 1.04 |
| ETHYLENE % | 1.99 | 2.21 |
| PROPANE % | 3.42 | 3.75 |
| PROPYLENE % | 5.18 | 7.09 |
| ISOBUTANE % | 8.89 | 9.25 |
| NBUTANE % | 2.23 | 2.41 |
| NBUTENES % | 3.28 | 4.07 |
| ISOBUTENE % | 1.08 | 2.22 |
| | | |
| **Ratios** | | |
| BUTENE / BUTANE | 0.39 | 0.54 |
| PROPYLENE / PROPANE | 1.51 | 1.89 |
| ISOBUTANE /NBUTANE | 3.99 | 3.84 |
| H₂ + C1 + C2 | 3.92 | 4.14 |
| ISOBUTENE / NBUTENES | 0.33 | 0.55 |
| ISOBUTENE / ISOBUTANE | 0.12 | 0.24 |
| H₂ + C1 + C2 / ISOBUTENE | 0.44 | 0.45 |
| C3 / C4 | 0.56 | 0.60 |
| ISOBUTENE + ISOBUTANE / TOTAL C4 | 0.64 | 0.64 |
| ISOBUTANE / NBUTANE | 0.08 | 0.14 |
| H₂ + C1 + C2 / ISOBUTENE + ISOBUTANE | 0.39 | 0.36 |
| ETHYLENE / ETHANE | 1.91 | 2.12 |
| ETHYLENE / PROPYLENE | 0.39 | 0.31 |
| | | |
| **Composition of the gasoline % by weight** | 75.81 | 77.63 |
| AROMATICS % | 11.85 | 9.13 |
| ISOPARAFFINS % | 6.90 | 5.62 |
| NAPHTHENES % | 3.43 | 3.71 |
| OLEFINS % | 2.02 | 3.91 |
| PARAFFINS % | 90.68 | 91.04 |
| RON | 86.31 | 86.80 |
| MON | 41.6 | 36.7 |
| RON barrel | 39.6 | 35.0 |
| MON barrel | 0.91 | 0.94 |
| ISOAMYLENES % | | |

### EXAMPLE 10:

The present example illustrates the use of a material prepared according to example 5 and activated by calcination in air at 580°C for 3 hours as a catalyst in alkylation of benzene with propylene.

An activated sample of the material prepared according to example 5 was made into cake form, selecting the particle size between 0.25 and 0.42 mm, in order to carry out the reaction. The zeolite (0.55 g) was diluted with silicon carbide (0.59 - 0.84 mm) in a ratio by weight of SiC/zeolite of 5. The diluted catalyst was introduced into a tubular steel reactor of diameter 1 cm and 100 mL.min⁻¹ of N₂ was passed under standard conditions at 150°C for 1.5 hours. The temperature was then lowered to 20°C and the N₂ stream was cut off. At this point, benzene was fed (1200 µL.min⁻¹) and the pressure was raised to 3.5 MPa. Once the pressure had reached 3.5 MPa, the temperature was raised to 125°C and the propylene started to be fed (270 µL.min⁻¹), the molar ratio of benzene/propylene being 3.4.

The results of converted propylene are presented in Table VII.

**Table VII: Conversion and selectivity in alkylation of benzene with propylene at 125°C, B/P = 3.4 mol.mol⁻¹, WHSV prop = 6 h⁻¹, P = 3.5 MPa for the catalyst Al-ITQ-22 prepared according to example 5.**

| Reaction time | Conversion (%) | Selectivity referred to propylene (%) | | | |
|---|---|---|---|---|---|
| (min) | | Cumene | DIPB | NPB | Others |
| 15 | 95.62 | 93.09 | 6.70 | 0.04 | 0.17 |
| 60 | 98.02 | 93.01 | 6.73 | 0.05 | 0.22 |
| 100 | 97.23 | 93.89 | 5.75 | 0.03 | 0.33 |
| 140 | 96.30 | 95.83 | 3.83 | 0.03 | 0.31 |
| 180 | 97.94 | 95.55 | 4.27 | 0.03 | 0.15 |

In this example, very high values of selectivity to cumene can be seen along with very low values of NPB and diisopropylbenzene (DIPB), less than those obtained using zeolite Beta as catalysts under the same reaction conditions. The results obtained with a commercial zeolite Beta of Si/Al = 13 supplied by Zeolyst (code CP811) are shown in Table VIII.

**Table VIII: Conversion and selectivity in alkylation of benzene with propylene at 125°C, B/P = 3.4 mol.mol⁻¹, WHSV prop = 6 h⁻¹, P = 3.5 MPa for a commercial catalyst of zeolite Beta (Si/Al = 13).**

| Reaction time | Conversion (%) | Selectivity referred to propylene (%) | | | |
|---|---|---|---|---|---|
| (min) | | Cumene | DIPB | NPB | Others |
| 20 | 98.07 | 91.35 | 8.45 | 0.04 | 0.16 |
| 90 | 97.36 | 92.41 | 7.35 | 0.05 | 0.19 |
| 160 | 98.54 | 90.84 | 8.85 | 0.05 | 0.26 |
| 210 | 99.65 | 93.28 | 6.49 | 0.05 | 0.18 |

From a comparison of the results presented in Tables VI and VIII, it can be seen that zeolite ITQ-22 displays greater selectivity to cumene and less to n-propylbenzene than zeolite Beta, which is the zeolitic catalyst currently most used for producing cumene.

### EXAMPLE 11:

The present example shows the influence of the reaction temperature on the conversion and selectivity for alkylation of benzene with propylene using the same catalyst as in example 10, the rest of the reaction conditions being the same as in example 10.

The results of the conversion with the reaction time are presented in Table IX.

**Table IX: Conversion and selectivity in alkylation of benzene with propylene at 150°C, B/P = 3.4 mol.mol⁻¹, WHSV prop = 6 h⁻¹ , P = 3.5 MPa for the catalyst Al-ITQ-22 prepared according to example 5.**

| Reaction time | Conversion (%) | Selectivity referred to propylene (%) | | | |
|---|---|---|---|---|---|
| (min) | | Cumene | DIPB | NPB | Others |
| 20 | 99.51 | 97.25 | 2.62 | 0.06 | 0.07 |
| 60 | 98.70 | 94.17 | 5.62 | 0.07 | 0.14 |
| 100 | 99.41 | 96.10 | 3.77 | 0.06 | 0.07 |
| 180 | 99.31 | 95.04 | 4.74 | 0.06 | 0.16 |

### EXAMPLE 12:

The present example illustrates the use of a material prepared according to example 5 and activated by calcination in air at 580°C for 3 hours as a catalyst in alkylation of benzene with ethylene.
An activated sample of the material prepared according to example 5 was made into cake form, selecting the particle size between 0.25 and 0.42 mm, in order to carry out the reaction. So, 0.55 g of the zeolite was diluted in 1.57 g of silicon carbide (0.59 - 0.84 mm). The diluted catalyst was introduced into a tubular steel reactor of diameter 1 cm. The activation of the catalyst was the same as in example 10. In the present example the reaction temperature was 220°C, with 2.90 mmoles of benzene and 0.30 mmoles of ethylene being fed, the pressure of the process being 3.5 MPa. The results obtained are presented in Table X.

**Table X: Conversion and selectivity in alkylation of benzene with ethylene at 220°C, B/E = 9.6 mol.mol⁻¹, WHSV ethyl = 1.0 h⁻¹, P = 3.5 MPa for the catalyst Al-ITQ-22 prepared according to example 5.**

| Reaction | Ethylene | Selectivity (%) | | | |
|---|---|---|---|---|---|
| t (min) | conversion (%) | Ethyl benzene | Butyl benzene | Diethyl benzene | Triethyl benzene |
| 30 | 99.40 | 98.20 | 0.09 | 1.71 | 0.00 |
| 120 | 99.20 | 98.10 | 0.10 | 1.80 | 0.00 |
| 210 | 99.10 | 97.47 | 0.16 | 2.37 | 0.00 |
| 300 | 99.18 | 98.54 | 0.09 | 1.36 | 0.01 |
| 420 | 99.52 | 97.67 | 0.12 | 2.19 | 0.02 |

### EXAMPLE 13:

This example illustrates the use of a sample Ti-ITQ-22 prepared according to example 6 and activated by calcination in air at 580°C for 3 hours, as a catalyst for the epoxidation reaction of 1-hexene with hydrogen peroxide.

1.420 grams of 1-hexene and 0.412 grams of hydrogen peroxide (35% in water) dissolved in 12.00 g of methanol are placed in a glass reactor. The reaction mixture is heated to 60°C and 0.100 grams of activated catalyst Ti-ITQ-22 are added. After 3 hours of reaction at this temperature with stirring, a 15% conversion of olefin is obtained with respect to the maximum possible, bearing in mind the olefin/oxidising agent molar ratio used in this example.

### EXAMPLE 14:

This example illustrates the use as a catalyst of a sample Ti-ITQ-22 prepared according to example 6 and activated by calcination in air at 580°C for 3 hours for the epoxidation reaction of 1-dodecene with hydrogen peroxide.

2.780 grams of 1-dodecene and 0.440 grams of hydrogen peroxide (35% in water) dissolved in 60.00 g of methanol are placed in a glass reactor. The reaction mixture is heated to 60°C and 0.100 grams of activated catalyst Ti-ITQ-22 are added. After 7 hours of reaction at this temperature with stirring, a 5% conversion of olefin is obtained with respect to the maximum possible, bearing in mind the olefin/oxidising agent molar ratio used in this example.

### EXAMPLE 15:

This example illustrates the use of a sample Ti-ITQ-22 prepared according to example 6 and activated by calcination in air at 580°C for 3 hours, as a catalyst for the epoxidation reaction of cyclohexene with terc-butyl-hydroperoxide.

9.270 grams of cyclohexane and 3.180 grams of terc-butyl-hydroperoxide are placed in a glass reactor. The reaction mixture is heated to 60°C and 0.600 grams of activated catalyst Ti-ITQ-22 are added. After 5 hours of reaction at this temperature with stirring, a 4% conversion of olefin is obtained with respect to the maximum possible, bearing in mind the olefin/oxidising agent molar ratio used in this example.

### EXAMPLE 16:

This example illustrates the use as a catalyst of a sample Sn-ITQ-22 prepared according to example 7 and activated by calcination in air at 580°C for 3 hours in the Baeyer-Villiger reaction between cyclohexanone and hydrogen peroxide. 0.063 grams of cyclohexanone and 0.078 grams of hydrogen peroxide (35% in water) dissolved in 1.520 g of dioxane are placed in a glass reactor. The reaction mixture is heated to 70°C and 0.025 grams of activated catalyst Sn-ITQ-22 are added. After 7 hours of reaction at this temperature with stirring, a 47% conversion of ketone is obtained with respect to the maximum possible, bearing in mind the ketone/oxidising agent molar ratio used in this example.

### EXAMPLE 17:

This example illustrates the use as a catalyst of a sample Sn-ITQ-22 prepared according to example 7 and activated by calcination in air at 580°C for 3 hours for the Baeyer-Villiger reaction between adamantanone and hydrogen peroxide.

0.250 grams of adamantanone and 0.259 grams of hydrogen peroxide (35% in water), dissolved in 1.530 g of dioxane, are placed in a glass reactor. The reaction mixture is heated to 90°C and 0.025 grams of activated catalyst Sn-ITQ-22 are added. After 7 hours of reaction at this temperature with stirring, a 22% conversion of ketone is obtained with respect to the maximum possible, bearing in mind the ketone/oxidising agent molar ratio used in this example.

## Claims

1. A microporous crystalline material of zeolitic nature which, in the calcined state and in the absence of defects in its crystal lattice manifested by the presence of silanols, has the empirical formula
***x* (H XO₂) : *y* YO₂ : *z* GeO₂ : (1 - *z*) SiO₂**
in which:
X is at least one chemical element of oxidation state +3, preferably selected from the group consisting of Al, Ga, B, Fe and Cr;
Y is at least one chemical element with oxidation state +4 other than Si and Ge, preferably selected from the group consisting of Ti, Sn and V,
**characterised in that**
*x* has a value less than 0.2, preferably less than 0.1 and can take the value zero,
*y* has a value less than 0.1, preferably less than 0.05 and can take the value zero,
*z* has a value less than 0.8, preferably between 0.005 and 0.5 and can take the value zero,
and **in that** the material, as synthesised, has an X-ray diffraction pattern with values of angle 2θ (degrees) and relative intensities (I/I₀) concordant with
| 2θ (degrees) ± 0.5 | Intensity (I/I₀) |
|---|---|
| 8.02 | vs |
| 8.43 | vs |
| 9.31 | m |
| 10.64 | vs |
| 20.26 | m |
| 21.81 | s |
| 22.53 | vs |
where
m is a medium relative intensity between 20 and 40%;
s is a strong relative intensity between 40 and 60%, and
vs is a very strong relative intensity between 60 and 100%.

2. A microporous crystalline material of zeolitic nature according to claim 1, **characterised in that**, in the calcined state, it has an X-ray diffraction pattern with values of angle 2θ (degrees) and relative intensities (I/I₀) concordant with
| 2θ (degrees) ± 0.5 | Intensity (I/I₀) |
|---|---|
| 6.97 | m |
| 8.00 | vs |
| 8.40 | vs |
| 9.28 | m |
| 10.62 | vs |
| 20.18 | m |
| 21.74 | m |
| 22.54 | vs |
where
m is a medium relative intensity between 20 and 40%;
s is a strong relative intensity between 40 and 60%, and
vs is a very strong relative intensity between 60 and 100%.

3. A method for synthesising the microporous crystalline material of claim 1 or 2 in which a reaction mixture containing
a source of SiO₂,
optionally a source of GeO₂,
optionally a source of other tetravalent element or elements Y, preferably Ti, V, Sn,
optionally a source of other trivalent element or elements X, preferably Al, B, Ga, Fe, Cr,
one or several sources of 1,5-bis (methylpyrrolidinium) pentane (R), and
water,
is subjected to heating with or without stirring at a temperature between 80 and 200°C, preferably between 130 and 200°C, until achieving crystallisation, **characterised in that** the reaction mixture has a composition in terms of molar ratios of oxides lying in the ranges
ROH/SiO₂ = 0.01-1.0, preferably 0.1-1.0
X₂O₃/SiO₂ = 0-0.1, preferably 0-0.05
YO₂/SiO₂ = 0-0.1, preferably 0-0.05
GeO₂/SiO₂ = 0-4, preferably 0.005-1
H₂O/SiO₂ = 1-50

4. A method according to claim 3, **characterised in that** the organic cation 1,5-bis (methylpyrrolidinium) pentane is added in dihydroxide form or in the form of a mixture of hydroxide and another salt, preferably a halide.

5. A method according to claim 4, **characterised in that** a quantity of crystalline material is added to the reaction mixture, preferably with the characteristics of the material of one of claims 1 and 2, as promoter of the crystallisation, said quantity lying in the range 0.01 to 20% by weight with respect to the total of inorganic oxides added, preferably between 0.05 and 10%.

6. Use of a microporous crystalline material of one of claims 1-2, **characterised in that** it is used as a component of a catalyst in a process selected among processes of cracking, hydrocracking, gentle hydrocracking of hydrocarbons and/or functionalised hydrocarbons, a process of isomerisation of light paraffins, a process of deparaffining or isodeparaffining, a process of alkylation of isoparaffins with olefins, a process of alkylation of aromatic compounds with an alkylating agent selected among olefins, alcohols, polyalkylated aromatics or mixtures thereof, an acylation process, and an oxidation process.

7. Use according to claim 6 in a catalytic cracking process of organic compounds wherein the catalyst includes the microporous cristalline material of one of claims 1 or 2, also referred to as zeolite ITQ-22 as sole zeolitic component embedded in a matrix, or zeolite ITQ-22 material together with at least one second zeolitic component embedded in a matrix.

8. Use according to claim 7 wherein the said at least one second zeolitic component is formed by zeolitic structures containing pores delimited by rings of 14 members, by rings of 12 members, by rings of 11 members, by rings of 10 members and mixtures thereof.

9. Use according to claim 7, wherein at least part of the catalyst particles comprise a second zeolitic component selected among one or more of the group comprising zeolite faujasite Y, ZSM-5 and zeolite Beta.

10. Use according to claim 7, wherein said catalyst is partially or wholly constituted by a single type of particle with the zeolite ITQ-22 material and said at least one second zeolitic component being present in the same particle.

11. Use according to claim 10, wherein the said second zeolitic component is zeolite faujasite Y under the form of one or more of its variants selected from the group consisting of zeolite faujasite Y, ultrastable zeolite faujasite Y, zeolite faujasite Y fully exchanged with rare earths, zeolite faujasite Y partially exchanged with rare earths, ultrastable zeolite faujasite Y fully exchanged with rare earths, ultrastable zeolite faujasite Y partially exchanged with rare earths and mixtures thereof, zeolite ITQ-22 being present in quantities between 0.1 and 60% by weight with respect to the total of zeolitic components, and zeolite faujasite Y in a quantity between 0.1 and 99.9% by weight with respect to the total of zeolitic components, the rest of the composition of the catalyst up to 100% being formed by the matrix.

12. Use according to claim 11, wherein at least part of the particles of the catalyst comprise:
- 0.1-40% by weight of the zeolitic material ITQ-22 with respect to the total weight of zeolitic components, and
- 0.1-99.9% by weight of the zeolite faujasite Y with respect to the total weight of zeolitic components.

13. Use according to claim 7, wherein said catalyst consists partially or wholly of at least two types of particle, with ITQ-22 and said at least one second zeolitic component being present in different particles.

14. Use according to claim 13, wherein the catalytic cracking catalyst comprises:
0.1-40% by weight of particles containing zeolite ITQ-22 with respect to the sum of zeolitic components, wherein each particle comprises 10-70% by weight of zeolite ITQ-22;
0.1-99.9% by weight of particles of conventional catalytic cracking catalyst based on zeolite faujasite Y, wherein the percentages are indicated with respect to the sum of zeolitic components.

15. Use according to claim 13, wherein said catalytic cracking catalyst in addition comprises particles containing zeolite ZSM-5, the composition of the catalyst being:
- zeolite faujasite Y in a quantity of at least 20% by weight with respect to the total weight of zeolitic components,
- quantity of zeolite ITQ-22 + ZSM-5 is a maximum of 80% by weight, with respect to the total weight of zeolitic components, and
- the ratio by weight between zeolite ZSM-5 and zeolite ITQ-22 lies between 10 and 0.

16. Use according to claim 7, whrein the matrix contains at least one binder selected among silica, silica-alumina, alumina, P₂O₅ and combinations thereof, and optionally also a conventional additive for catalytic cracking.

17. Use according to claim 7, wherein any of the zeolitic components also contain a maximum of 8% by weight of phosphorus.

18. Use according to claim 7, wherein any of the zeolitic components are exchanged with ions selected among divalent ions, trivalent ions, divalent and trivalent ions, and rare earths.

19. Use according to claim 7, wherein any of the zeolitic components comprise compounds of vanadium introduced in a post-synthesis stage.

20. Use according to claim 7, wherein any of the zeolitic components comprise cerium introduced in a post-synthesis stage.

21. Use according to claim 7, wherein the catalyst is formed of particles with a size between 20 and 400 micrometres.

22. Use according to claim 7, wherein the catalytic process is selected between a catalytic cracking process in fluid bed (FCC) and a deep catalytic cracking (DCC) process.

23. Use according to claim 6, wherein the organic compounds are hydrocarbons derived from fractions of natural or synthetic petroleum.

24. Use of zeolite ITQ-22 according to claim 6 in a process for the alkylation of aromatic compounds, wherein an alkylating agent selected among an olefin, an alcohol, a polyalkylated aromatic compound and mixtures thereof is made to react under alkylation conditions with a starting aromatic compound in the presence of a catalyst, said catalyst being ITQ-22.

25. Use according to claim 24, wherein the starting aromatic compound is selected from the group consisting of benzene, naphthalene, anthracene, phenanthrene and substituted derivatives thereof.

26. Use according to claim 24, wherein the starting aromatic compound is selected among alkylbenzene, alkylanthracene, alkylphenanthrene, hydroxybenzene, hydroxynaphthalene, hydroxyanthracene, hydroxyphenanthrene, alcoxybenzene, alcoxynaphthalene, alcoxyanthracene and alcoxyphenanthrene.

27. Use according to claim 24, wherein the alkylating agent is selected among an olefin, an alcohol and mixtures thereof and said olefin and said alcohol contain from 2 to 20 carbon atoms.

28. Use according to claim 26, wherein the alkylating agent is a polyalkylated aromatic compound, the starting aromatic compound is a non-alkylated aromatic compound, and in which during the alkylation at least one alkyl group is transferred from the polyalkylated aromatic compound to the starting aromatic compound.

29. Use according to claim 28 wherein said polyalkylated aromatic compound contains at least one alkyl group which comprises from 2 up to 20 carbon atoms.

30. Use according to claim 28 wherein the starting aromatic compound is selected among benzene, naphthalene, anthracene, phenanthrene, substituted benzene, substituted naphthalene, substituted anthracene and substituted phenanthrene.

31. Use according to claim 28 wehrein the polyalkylated aromatic compound is polyisopropylbenzene and the starting aromatic compound is benzene.

32. Use according to claim 24, wherein the starting aromatic compound is benzene, the alkylating agent is propylene and in which the alkylation procedure produces cumene as alkylated aromatic compound.

33. Use according to claim 24, wherein the alkylation reaction is conducted at a reaction temperature of between 60 and 350°C.

34. Use according to claim 24, wherein the alkylation reaction is conducted at a pressure between 1.4 and 7.0 MPa.

35. Use according to claim 24, wherein the alkylating agent and the starting aromatic compound are present in a proportion of between 2 and 20, in the presence of the catalyst.

36. Use according to claim 24, wherein the starting aromatic compound is benzene and the alkylating agent is propylene and the alkylated aromatic compound that is obtained is cumene; the alkylation reaction is carried out at a reaction temperature between 60 and 350°C; the pressure at which the alkylation reaction is carried out lies between 1.4 and 7.0 MPa; the spatial velocity (WHSV) of reagents lies between 0.2 and 10 hours⁻¹ and the benzene/propylene molar ratio is between 2 and 20.

37. Use according to claim 6, **characterised in that** the material contains Ti and is used as a catalyst in a process selected among selective oxidation processes of organic compounds using an oxidising agent selected among H₂O₂ or peroxides, hydroperoxides or organic peracids.

38. Use according to claim 6, **characterised in that** the material contains Sn and is used as a catalyst in a process selected among oxidation processes of the Baeyer-Villiger type.

39. Use according to claim 6, **characterised in that** the material is used as a catalyst in a process selected among oxidation processes of the Meerwein-Pondorf-Verley type.

40. Use according to claim 6, **characterised in that** the material is used as a catalyst in a process selected among hydroisomerisation processes of olefins, alkylation of olefins with isoparaffins and alkylation of aromatics with olefins or alcohols.

## Patentansprüche

1. Mikroporöses kristallines Material von zeolithische Beschaffenheit, welches im kalzinierten Zustand und in Abwesenheit von Defekten in seinem Kristallgitter, offenbart durch die Anwesenheit von Silanolen, die empirische Formel
***x* (H XO₂) : *y* YO₂ : *z* GeO₂ (1 - *z*) SiO₂**
hat, worin
x mindestens ein chemisches Element mit Oxidations zustand +3 ist, vorzugsweise ausgewählt aus der Gruppe bestehend aus Al, Ga, B, Fe und Cr;
Y mindestens ein anderes chemisches Element als Si und Ge mit Oxidationszustand +4 ist, vorzugsweise ausgewählt aus der Gruppe bestehend aus Ti, Sn und V,
**dadurch gekennzeichnet, dass**
x einen Wert kleiner als 0,2, vorzugsweise kleiner als 0,1 hat und den Wert null annehmen kann,
*y* einen Wert kleiner als 0,1, vorzugsweise kleiner als 0,05 hat und den Wert null annehmen kann,
*z* einen Wert kleiner als 0,8, vorzugsweise zwischen 0,005 und 0,5 hat und den Wert null annehmen kann,
und **dadurch**, dass das Material, wie synthetisiert, ein Röntgenbeugungsdiagramm mit Werten von Winkel 2θ (Grad) und relativen Intensitäten (I/I₀) übereinstimmend mit
| 2θ (Grad) ± 0,5 | Intensität (I/I₀) |
|---|---|
| 8,02 | vs |
| 8,43 | vs |
| 9,31 | m |
| 10,64 | vs |
| 20,26 | m |
| 21,81 | s |
| 22,53 | vs |
m für eine mittlere relative Intensität zwischen 20 und 40% steht;
s für eine starke relative Intensität zwischen 40 und 60% steht und
vs für eine sehr starke relative Intensität zwischen 60 und 100% steht.

2. Mikroporöses kristallines Material von zeolithischer Beschaffenheit gemäß Anspruch 1, **dadurch gekennzeichnet, dass** es im kalzinierten Zustand ein Röntgenbeugungsdiagramm mit Werten von Winkel 2θ (Grad) und relativen Intensitäten (I/I₀) übereinstimmend mit
| 2θ (Grad) ± 0,5 | Intensität (I/I₀) |
|---|---|
| 6,97 | m |
| 8,00 | vs |
| 8,40 | vs |
| 9,28 | m |
| 10,62 | vs |
| 20,18 | m |
| 21,74 | m |
| 22,54 | vs |
hat, wo
m für eine mittlere relative Intensität zwischen 20 und 40% steht;
s für eine starke relative Intensität zwischen 40 und 60% steht und
vs für eine sehr starke relative Intensität zwischen 60 und 100% steht.

3. Verfahren zum Synthetisieren des mikroporösen kristallinen Materials nach Anspruch 1 oder 2, wobei ein Umsetzungsgemisch, welches
eine Quelle für SiO₂,
gegebenenfalls eine Quelle für GeO₂,
gegebenenfalls eine Quelle für andere (s) tetravalente(s) Element oder Elemente Y, vorzugsweise Ti, V, Sn,
gegebenenfalls eine Quelle für anders(s) trivalente(s) Element oder Elemente X, vorzugsweise Al, B, Ga, Fe, Cr,
ein oder mehrere Quellen für 1,5-Bis(methylpyrrolidinium)-pentan (R) und
Wasser
enthält, Erhitzen mit oder ohne Rühren bei einer Temperatur zwischen 80 und 200°C, vorzugsweise zwischen 130 und 200°C unterworfen wird, bis Kristallisation erreicht wird, **dadurch gekennzeichnet, dass** das Umsetzungsgemisch eine Zusammensetzung bezüglich Molverhältnisse von Oxiden hat, welche in den Bereichen
ROH/SiO₂ = 0,01-1,0, vorzugsweise 0,1-1,0
X₂O₃/SiO₂ = 0-0,1, vorzugsweise 0-0,05
YO₂/SiO₂ = 0-0,7, vorzugsweise 0-0,05
GeO₂/SiO₂ = 0-4, vorzugsweise 0,005-1
H₂O/SiO₂ = 1-50
liegt.

4. Verfahren gemäß Anspruch 3, **dadurch gekennzeichnet, dass** das organische Kation 1,5-Bis(methylpyrrolidinium)pentan in Dihydroxidform oder in Form eines Gemisches aus Hydroxyd und einem anderen Salz, vorzugsweise einem Halogenid zugegeben wird.

5. Verfahren gemäß Anspruch 4, **dadurch gekennzeichnet, dass** eine Menge an kristallinem Material zum Ümsetzungsgemisch vorzugsweise mit den Kennzeichen des Materials nach einem von Ansprüchen 1 und 2 als Beschleuniger der Kristallisation zugegeben wird, wobei besagte Menge im Bereich 0,01 bis 20 Gew.-% bezogen auf die Gesamtmenge der zugegebenen anorganischen Oxide, vorzugsweise zwischen 0,05 und 10% liegt.

6. Verwendung eines mikroporösen kristallinen Materials nach einem von Ansprüchen 1-2, **dadurch gekennzeichnet, dass** es als Bestandteil eines Katalysators in einem Verfahren, ausgewählt aus Verfahren des Krackens, Hydrokrackens, sanften Hydrokrackens von Kohlenwasserstoffen und/oder funktionalisierten Kohlenwasserstoffen, einem Verfahren der Isomerisation von leichten Paraffinen, einem Verfahren des Entparaffinierens oder Isoentparaffinierens, einem Verfahren der Alkylierung von Isoparaffinen mit Olefinen, einem Verfahren der Alkylierung von aromatischen Verbindungen mit einem Alkylierungsmittel, ausgewählt aus Olefinen, Alkoholen, polyalkylierten aromatischen Substanzen oder Gemischen daraus, einem Acylierungsverfahren und einem Oxidationsverfahren verwendet wird.

7. Verwendung gemäß Anspruch 6 in einem katalytische Krackverfahren von organischen Verbindungen, wobei der Katalysator das mikroporöse kristalline Material nach einem der Ansprüche 1 bis 2, auch als Zeolith ITQ-22 bezeichnet, als einzigen zeolithischen Bestandteil, eingebettet in einer Matrix, oder Zeolith ITQ-22-Material zusammen mit mindestens einem zweiten zeolithischen Bestandteil, eingebettet in einer Matrix, einschließt.

8. Verwendung gemäß Anspruch 7, wobei der besagte mindestens eine zweite zeolithische Bestandteil gebildet ist durch zeolithische Strukturen, welche Poren, abgegrenzt durch Ringe aus 14 Gliedern, durch Ringe aus 12 Gliedern, durch Ringe aus 11 Gliedern, durch Ringe aus 10 Gliedern und Gemischen daraus, enthalten.

9. Verwendung gemäß Anspruch 7, wobei mindestens ein Teil der Katalysatorpartikel einen zweiten zeolithischen Bestandteil umfassen, ausgewählt aus einem oder mehreren der Gruppe, welche Zeolith Faujasit Y, ZSM-5 und Zeolith-Beta umfasst.

10. Verwendung gemäß Anspruch 7, wobei besagter Katalysator teilweise oder ganz durch einen einzelnen Partikeltyp mit dem Zeolith ITQ-22 Material gebildet ist und besagter mindestens eine zweite zeolithische Bestandteil im gleichen Partikel vorhanden ist.

11. Verwendung gemäß Anspruch 10, wobei der besagte zweite zeolithische Bestandteil Zeolith Faujasit Y unter der Form von einer oder mehreren seiner Varianten ist, ausgewählt aus der Gruppe bestehend aus Zeolith Faujasit Y, ultrastabilem Zeolith Faujasit Y, Zeolith Faujasit Y, vollständig ausgetauscht mit seltenen Erden, Zeolith Faujasit Y, teilweise ausgetauscht mit seltenen Erden, ultrastabilem Zeolith Faujasit Y, vollständig ausgetauscht mit seltenen Erden, ultrastabilem Zeolith Faujasit Y, teilweise ausgetauscht mit seltenen Erden, und Gemischen daraus, wobei Zeolith ITQ-22 in Mengen zwischen 0,1 und 60 Gew.-% bezogen auf die Gesamtmenge an zeolithischen Bestandteilen vorhanden ist und Zeolith Faujasit Y in einer Menge zwischen 0,1 und 99,9 Gew.-% bezogen auf die Gesamtmenge an zeolithischen Bestandteilen vorhanden ist, wobei der Rest den Zusammensetzung des Katalysators bis zu 100% durch die Matrix gebildet wird.

12. Verwendung gemäß Anspruch 11, wobei mindestens ein Teil der Partikel des Katalysators umfasst:
- 0,1-40 Gew.-% des zeolithischen Materials ITQ-22 bezogen auf das Gesamtgewicht an zeolithische Bestandteile, und
- 0,1-99,9 Gew.-% des Zeoliths Faujasit Y bezogen auf das Gesamtgewicht an zeolithischen Bestandteilen.

13. Verwendung gemäß Anspruch 7, wobei besagter Katalysator teilweise oder ganz, aus mindestens zwei Partikeltypen besteht, wobei ITQ-22 und besagter mindestens ein zweiter zeolithischer Bestandteil in verschiedenen Partikeln vorhanden sind.

14. Verwendung gemäß Anspruch 13, wobei der katalytische Krackkatalysator umfasst:
0,1-40 Gew.-% an Partikeln, welche Zeolith ITQ-22 enthalten, bezogen auf die Summe an zeolithischen Bestandteilen, wobei jeder Partikel 10-70 Gew.-% Zeolith ITQ-22 umfasst;
0,1-99,9 Gew.-% an Partikeln aus konventionellem katalytischen Krackkatalysator, basierend auf Zeolith Faujasit Y, wobei die Prozentsätze bezogen auf die Summe an zeolithischen Bestandteilen angezeigt werden.

15. Verwendung gemäß Anspruch 13, wobei besagter katalytischer Krackkatalysator zusätzlich Partikel umfasst, welche Zeolith ZSM-5 enthalten, wobei die Zusammensetzung des Katalysators ist:
- Zeolith Faujasit Y in einer Menge von mindestens 20 Gew.-bezogen auf das Gesamtgewicht an zeolithischen Bestandteilen,
- die Menge an Zeolith ITQ-22 + ZSM-5 maximal 80 Gew.-% ist, bezogen auf das Gesamtgewicht an zeolithischen Bestandteilen, und
- das Gewichtsverhältnis zwischen Zeolith ZSM-5 und Zeolith TTQ-22 zwischen 10 und 0 liegt.

16. Verwendung gemäß Anspruch 7, wobei die Matrix mindestens ein Bindemittel enthält, ausgewählt aus Siliciumdioxid, Siliciumdioxid-Aluminiumoxid, Aluminiumoxid, P₂O₅ und Kombinationen daraus, und gegebenenfalls ebenfalls ein konventionelles Zusatzmittel für katalytisches Kracken.

17. Verwendung gemäß Anspruch 7, wobei irgendwelche der zeolitischen Bestandteile auch maximal 8 Gew.-% Phosphor enthalten.

18. Verwendung gemäß Anspruch 7, wobei irgendwelche der zeolithischen Bestandteile mit Ionen ausgetauscht sind, ausgewählt aus zweiwertigen Ionen, dreiwertigen Ionen, zweiwertigen und dreiwertigen Ionen und seltenen Erden.

19. Verwendung gemäß Anspruch 7, wobei irgendwelche der zeolithischen Bestandteile Verbindungen aus Vanadium, eingeführt in einer Postsynthesestufe, umfassen.

20. Verwendung gemäß Anspruch 7, wobei irgendwelche der zeolithischen Bestandteile Cer, eingeführt in einer Postsynthesestufe, umfassen.

21. Verwendung gemäß Anspruch 7, wobei der Katalysator aus Partikeln mit einer Größe zwischen 20 und 400 Mikrometern gebildet ist.

22. Verwendung gemäß Anspruch 7, wobei das katalytische Verfahren ausgewählt wird zwischen einem katalytischen Krackverfahren im Fließbett (FCC) und einem tiefen katalytisches Krackverfahren (DCC).

23. Verfahren gemäß Anspruch 6, wobei die organischen Verbindungen Kohlenwasserstoffe sind, abgeleitet von Fraktionen von natürlichcm oder synthetischem Petroleum.

24. Verwendung von Zeolith ITQ-22 gemäß Anspruch 6 in einem Verfahren zur Alkylierung von aromatischen Verbindungen, wobei ein Alkylierungsmittel, ausgewählt aus einem Olefin, einem Alkohol, einer polyalkylierten aromatischen Vebindung und Gemischen daraus, veranlasst wird, sich unter Alkylierungsbedingungen mit einer ausgehenden aromatischen Verbindung in Anwesenheit eines Katalysators umzusetzen, wobei besagter Katalysator ITQ-22 ist.

25. Verwendung gemäß Anspruch 24, wobei die ausgehende aromatische Verbindung ausgewählt wird aus der Gruppe bestehend aus Benzol, Naphthalin, Anthracen, Phenanthren und substituierten Derivaten davon.

26. Verwendung gemäß Anspruch 24, wobei die ausgehende aromatische Verbindung ausgewählt wird aus Alkylbenzol, Alkylanthracen, Alkylphenanthren, Hydroxybenzol, Hydroxynaphthalin, Hydroxyanthracen, Hydroxyphenanthren, Alkoxybenzol, Alkoxynaphthalin, Alkoxyanthracen und Alkoxyphenanthren.

27. Verwendung gemäß Anspruch 24, wobei das Alkylierungsmittel ausgewählt wird aus einem Olefin, einem Alkohol und Gemischen daraus und besagtes Olefin und besagter Alkohol von 2 bis 20 Kohlenstoffatome entfalten.

28. Verwendung gemäß Anspruch 26, wobei das Alkylierungsmittel eine polyalkylierte aromatische Verbindung ist, die ausgehende aromatische Verbindung eine nicht-alkylierte aromatische Verbindung ist, und wobei während der Alkylierung mindestens eine Alkylgruppe von der polyalkylierten aromatischen Verbindung zur ausgehenden aromatischen Verbindung übertragen wird.

29. Verwendung gemäß Anspruch 28, wobei besagte polyalkylierte aromatische Verbindung mindestens eine Alkylgruppe enthält, welche von 2 bis 20 Kohlenstoffatome umfasst.

30. Verwendung gemäß Anspruch 28, wobei die ausgehende aromatische Verbindung ausgewählt ist aus Benzol, Naphthalin, Anthracen, Phenanthren, substituiertem Benzol, substituiertem Naphthalin, substituiertem Anthracen und substituiertem Phenanthren.

31. Verwendung gemäß Anspruch 28, wobei die polyalkylierte aromatische Verbindung Polyisopropylbenzol ist und die ausgehende aromatische Verbindung Benzol ist.

32. Verbindung gemäß Anspruch 24, wobei die ausgehende aromatische Verbindung Benzol ist, das Alkylierungsmittel Propylen ist, und wobei das Alkylierungsverfahren Cumol als alkylierte aromatische Verbindung herstellt.

33. Verwendung gemäß Anspruch 24, wobei die Alkylierungsumsetzung bei einer Umsetzungstemperatur von zwischen 60 und 350°C durchgeführt wird.

34. Verwendung gemäß Anspruch 24, wobei die Alkylierungsumsetzung bei einem Druck zwischen 1,4 und 7,0 MPa durchgeführt wird.

35. Verwendung gemäß Anspruch 24, wobei das Alkylierungsmittel und die ausgehende aromatische Verbindung in einer Proportion von zwischen 2 und 20 in Anwesenkreit des Katalysators vorhanden sind.

36. Verwendung gemäß Anspruch 24, wobei die ausgehende aromatische Verbindung Benzol ist und das Alkylierungsmittel Propylen ist und die alkylierte aromatische Verbindung, die erhalten wird, Cumol ist; die Alkyllerungsumsetzung bei einer Umsetzungstemperatur zwischen 60 und 350°C durchgeführt wird; der Druck, bei welchem die Alkylierungsumsetzung durchgeführt wird, zwischen 1,4 und 7,0 MPa liegt; die Raumgeschwindigkeit (WHSV) von Reagenzien zwischen 0,2 und 10 Stunden⁻¹ liegt und das Molverhältnis Benzol/Propylen zwischen 2 und 20 ist.

37. Verwendung gemäß Anspruch 6, **dadurch gekennzeichnet, dass** das Material Ti enthält und als Katalysator in einem Verfahren, ausgewählt aus selektiven Oxidationsverfahren von organischen Verbindungen unter Verwendung eines Oxidationsmittels, ausgewählt aus H₂O₂ oder Peroxiden, Hydroperoxiden oder organischen Persäuren, verwendet wird.

38. Verwendung gemäß Anspruch 6, **dadurch gekennzeichnet, dass** das Material Sn enthält und als Katalysator in einem Verfahren, ausgewählt aus Oxidationsverfahren des Baeyer-Villiger-Typs verwendet wird.

39. Verwendung gemäß Anspruch 6, **dadurch gekennzeichnet, dass** das Material als Katalysator in einem Verfahren, ausgewählt aus Oxidationsverfahren des Meerwein-Pondorf-Verley-Typs verwendet wird.

40. Verwendung gemäß Anspruch 6, **dadurch gekennzeichnet, dass** das Material als Katalysator in einem Verfahren, ausgewählt aus Hydroisomerisierungsverfahren von Olefinen, Alkylierung von Olefinen mit Isoparaffinen und Alkylierung von aromatischen Substanzen mit olefinen und Alkoholen, verwendet wird.

## Revendications

1. Matériau cristallin microporeux de nature zéolitique, qui à l'état calciné et en l'absence de défauts dans son réseau cristallin mis en évidence par la présence de silanols, a la formule empirique :
x (H XO₂) ; y YO₂ : z GeO₂ : (1-z) SiO₂,
dans laquelle :
X est au moins un élément chimique d'état d'oxydation +3, de préférence choisi parmi le groupe consistant en Al, Ga, B, Fe et Cr ;
Y est au moins un élément chimique d'état d'oxydation +4, autre que Si et Ge, de préférence choisi parmi le groupe consistant en Ti, Sn et v ;
**caractérisé en ce que**
x a une valeur inférieure à 0,2, de préférence inférieure à 0,1 et peut prendre la valeur zéro,
y a une valeur inférieure à 0,1, de préférence inférieure à 0,05 et peut prendre la valeur zéro,
z a une valeur inférieure à 0, 8, de préférence située dans l'intervalle allant de 0,005 à 0,5 et peut prendre la valeur zéro,
et **en ce que** le matériau, tel que synthétisé, a un motif de diffraction des rayons X avec des valeurs de l'angle 2θ (degrés) et des intensités relatives (I/I₀) correspondant à
| 2θ (degrés) ± 0,5 | Intensité (I/I₀) |
|---|---|
| 8,02 | vs |
| 8,43 | vs |
| 9,31 | m |
| 10,64 | vs |
| 20,26 | m |
| 21,81 | s |
| 22,53 | vs |
où
m est une intensité relative moyenne allant de 20 à 10% ;
s est une intensité relative importante allant de 40 à 60%, et
vs est une intensité relative très importante allant de 60 à 100%.

2. Matériau cristallin microporeux de nature zéolitique selon la revendication 1, **caractérisé en ce que**, à l'étal calciné, il a un motif de diffraction des rayons X avec des valeurs de l'angle 2θ (degrés) et des intensités relatives (I/I₀) correspondant à
| 2θ (degrés) ± 0,5 | Intensité (I/I₀) |
|---|---|
| 6,97 | m |
| 8,00 | vs |
| 8,40 | vs |
| 9,28 | m |
| 10,52 | vs |
| 20,18 | m |
| 21,74 | m |
| 22,54 | vs |
où
m est une intensité relative moyenne allant de 20 à 40% ;
s est une intensité relative importante allant de 40 à 60%, et
vs est une intensité relative très importante allant de 60 à 100%.

3. Procédé de synthèse du matériau cristallin microporeux selon la revendication 1 ou 2, dans lequel un mélange réactionnel contenant
une source de SiO₂,
le cas échéant, une source de GeO₂,
le cas échéant, une source d'un autre élément tétravalent ou des éléments Y, de préférence Ti, V, Sn,
le cas échéant, une source d'un autre élément trivalent ou des éléments X, de préférence Al, B, Ga, Fe, Cr,
une ou plusieurs sources de 1,5-bis(méthylpyrrolidinium)pentane (R), et
de l'eau,
est soumis à un chauffage avec ou sans agitation à une température située dans l'intervalle allant de 80 à 200°C, de préférence allant de 130 à 200°C, jusqu'à ce que l'on ait atteint la cristallisation, **caractérisé en ce que** le mélange rédactionnel a une composition en termes de rapports molaires d'oxydes se situant dans les plages :
ROH/SiO₂ = 0,01-10, de préférence 0,1-1,0,
X₂O₃/SiO₂ - 0-0,1, de préférence 0-0,05,
YO₂/SiO₂ = 0-0,1, de préférence 0-0,05,
GeO₂/SiO₂ = 0-4, de préférence 0,005-1,
HO₂/SiO₂ = 1-50.

4. Procédé selon la revendication 3, **caractérisé en ce que** le cation organique 1,5-bis-(méthylpyrrolidinium)pentane est ajouté sous forme de dihydroxyde ou sous forme d'un mélange d'hydroxyde et d'un autre sel, de préférence un halogénure.

5. Procédé selon la revendication 4, **caractérisé en ce que** la quantité du matériau cristallin est ajoutée au mélange réactionnel, de préférence avec les caractéristiques du matériau de l'une des revendications 1 et 2, comme promoteur de la cristallisation, ladite quantité se situant dans l'intervalle allant de 0,01 à 20% en poids par rapport au total des oxydes inorganiques ajoutés, de préférence allant de 0,05 à 10%.

6. Utilisation d'un matériau cristallin microporeux selon l'une des revendications 1-2,
**caractérisé en ce qu'**il est utilisé comme composant d'un catalyseur dans un procédé choisi parmi les procédés de craquage, d'hydrocraquage, d'hydrocraquage léger des hydrocarbures et/ou d'hydrocarbures fonctionnalisés, un procédé d'isomérisation de paraffines légères, un procédé de déparaffinage ou d'isodéparaffinage, un procédé d'alcoylation d'isoparaffines avec des oléfines, un procédé d'alcoylation de composés aromatiques avec un agent d'alcoylation choisi parmi les oléfines, les alcools, des aromatiques polyalcoylés ou leurs mélanges, un procédé d'acylation et un procédé d'oxydation.

7. Utilisation selon la revendication 6 dans un procédé catalytique de craquage de composés organiques, ou le catalyseur comprend le matériau cristallin microporeux selon l'une des revendications 1 et 2, désigné également zéolite ITQ-22 comme seul composé zéolitique piégé dans une matrice, ou un matériau zéolitique ITQ-22 avec au moins un deuxième composé zéolitique, piégés dans une matrice.

8. Utilisation selon la revendication 7, où ledit au moins un deuxième composé zéolitique est formé de structures zéolitiques contenant des pores délimités par des cycles de 14 éléments, par des cycles de 12 éléments, par, des cycles de 11 éléments, par des cycles de 10 éléments et leurs mélanges.

9. Utilisation selon la revendication 7, où au moins un partie des particules de catalyseur comprennent un deuxième composé zéolitique choisi parmi l'un ou plusieurs du groupe comprenant la zéolite faujasite Y, le ZSM-S et la zéolite Bêta.

10. Utilisation selon la revendication 7, où ledit catalyseur est partiellement ou complètement constitué d'un seul type de particule avec le matériau zéolitique ITQ-22 et ledit au moins deuxième composé zéolitique est présent dans la même particule.

11. Utilisation selon la revendication 10, où ledit deuxième composé zéolitique est la zéolite faujasite Y sous la forme d'un ou de plusieurs de ses variants, choisis parmi le groupe consistant en la zéolite faujasite Y, la zéolite faujasite Y ultrastable, la zéolite faujasite Y complètement échangée avec des terres rares, la zéolite faujasite Y partiellement échangée avec des terres rares, la zéolite faujasite Y ultrastable complètement échangée avec de terres rares, la zéolite faujasite Y ultrastable partiellement échangée avec de terres rares, et leurs mélange, la zéolite ITQ-22 étant présente en des quantités allant de 0,1 à 60% en poids par rapport au total des composés zéolitiques, et la zéolite faujasite Y en une quantité allant de 0,1 a 99,9% en poids par rapport au total des composés zéolitiques, le reste de la composition de catalyseur pour faire 100%, étant formé de la matrice.

12. Utilisation selon la revendication 11, où au moins une partie des particules du catalyseur, comprend :
- 0,1-40% en poids du matériau zéolitique ITQ-22 par rapport au poids total des composés zéolitiques, et
- 0,1-99,9% en poids de la zéolite faujasite Y par rapport au poids total des composés zéolitiques.

13. Utilisation selon la revendication 7, où ledit catalyseur consiste partiellement ou complètement en au moins deux types de particule, ITQ-22 et ledit au moins deuxième composé zéolitique étant présents dans des particules différentes.

14. Utilisation selon la revendication 13, où le catalyseur de craquage catalytique comprend :
- 0,1-40% en poids de particules contenant la zéolite TTQ-22 par rapport à la somme des composés zéolitiques, chaque particule comprenant 10-70% en poids de la zéolite ITQ-22, et
- 0,1-99,9% en poids de particules du catalyseur classique de craquage catalytique à base de la zéolite faujasite Y, les pourcentages étant données par rapport à la somme des composés zéolitiques.

15. Utilisation selon la revendication 13, où ledit catalyseur de craquage catalytique comprend en outre, des particules contenant la zéolite ZSM-5, la composition du catalyseur étant :
- la zéolite faujasite Y en une quantité d'au moins 20% en poids par rapport au poids total des composés zéolitique,
- la quantité de zéolite ITQ-22 + ZSM-5 est d'au maximum 80% en poids, par rapport au poids total des composés zéolitiques, et
- le rapport pondéral entre la zéolite ZSM-5 et la zéolite ITQ-22 se situe dans l'intervalle allant de 10 à 0.

16. Utilisation selon la revendication 7, où la matrice contient au moins un liant choisi parmi, la silice, la silice-alumine, l'alumine, P₂O₅ et leurs combinaisons, et le cas échéant également, un additif classique pour le craquage catalytique.

17. Utilisation selon la revendication 7, où l'un quelconque des composés zéolitiques contient également, un maximum de 8% en poids de phosphore.

18. Utilisation selon la revendication 7, où l'un quelconque des composés zéolitiques a été échangé avec des ions choisis parmi des ions bivalents, des ions trivalent, des ions bivalents et trivalents, et des terres rares.

19. Utilisation selon la revendication 7, où l'un quelconque des composés zéolitiques comprend des composés du vanadium, introduits dans une étape ultérieure à la synthèse.

20. Utilisation selon la revendication 7, où l'un quelconque des composés zéolitiques comprend du cérium introduit dans une étape ultérieure à la synthèse.

21. Utilisation selon la revendication 7, où le catalyseur est formé de particules ayant une taille située dans l'intervalle allant de 20 à 400 µm.

22. Utilisation selon la revendication 7, où le procédé catalytique est choisi parmi un procédé de craquage catalytique en lit fluide (FCC) et un procédé de craquage catalytique « deep » (DCC).

23. Utilisation selon la revendication 6, où les composés organiques sont des hydrocarbures dérivés de fractions de pétrole naturel ou synthétique.

24. Utilisation de la zéolite ITQ-22 selon la revendication 6, dans un procédé d'alcoylation de composés aromatiques, où un agent d'alcoylation choisi parmi une oléfine, un alcool, un composé aromatique polyalcoylé et leurs mélange, est préparé pour réagir dans des conditions d'alcoylation, avec un composé aromatique de départ, en présence d'un catalyseur, ledit catalyseur étant l'ITQ-22.

25. Utilisation selon la revendication 24, ou le composé aromatique de départ est choisi parmi le groupe consistant en le benzène, le naphtalène, l'anthracène, le phénanthrène et leurs dérivés substitués.

26. Utilisation selon la revendication 24, où le composé aromatique de départ est choisi parmi un alcoylbenzène, un alcoylanthracène, un alcoylphénanthrène, l'hydroxybenzène, l'hydroxynaphtalène, l'hydroxyanthracène, l'hydroxyphénanthrène, un alcoxybenzène, un alcoxynaphtalène, un alcoxyanthracène et un alcoxyphénanthrène.

27. Utilisation selon la revendication 24, où l'agent alcoylant est choisi parmi une oléfine, un alcool et leurs mélanges, ladite oléfine et ledit alcool contenant 2 à 20 atomes de carbone.

28. Utilisation selon la revendication 26, où l'agent alcoylant est un composé aromatique polyalcoylé, le composé aromatique de départ est un composé aromatique non alcoylé, et dans lequel, pendant l'alcoylation, au moins un radical alcoyle est transféré du composé aromatique polyalcoylé au composé aromatique de départ.

29. Utilisation selon la revendication 28, où ledit composé aromatique polyalcoylé contient au moins un radical alcoyle, qui comprend 2 à 20 atomes de carbone.

30. Utilisation selon la revendication 28, où le composé aromatique de départ est choisi parmi le benzène, le naphtalène, l'anthracène, le phénanthrène, un benzène substitué, un naphtalène substitué, un anthracène substitué et un phénanthrène substitué.

31. Utilisation selon la revendication 28, où le composé aromatique polyalcoylé est le polyisopropylbenzène et le composé aromatique de départ est le benzène.

32. Utilisation selon la revendication 24, où le composé aromatique de départ est le benzène, l'agent d'alcoylation est le propylène et où la procédure d'alcoylation conduit au cumène comme composé aromatique alcoylé.

33. Utilisation selon la revendication 24, où la réaction d'alcoylation est réalisée à une température de réaction allant de 60 à 350°C.

34. Utilisation selon la revendication 24, où la réaction d'alcoylation est réalisée à une pression allant de 1,4 à 7,0 MPa.

35. Utilisation selon la revendication 24, où l'agent d'alcoylation et le composé aromatique de départ sont présentes en une proportion allant de 2 à 20, en présence du catalyseur.

36. Utilisation selon la revendication 24, où le composé aromatique de départ est le benzène, l'agent d'alcoylation est le propylène et le composé aromatique alcoylé, qui est obtenu, est le cumène ; la réaction d'alcoylation est réalisée à une température de réaction allant de 60 à 350°C ; la pression à laquelle la réaction d'alcoylation est réalisée, se situe dans l'intervalle allant de 1,4 à 7,0 MPa ; la vitesse spatiale (WHSV) des réactifs se situe dans l'intervalle allant de 0, 2 à 10 h⁻¹ et le rapport molaire benzène /propylène se situe dans l'intervalle allant de 2 à 20.

37. Utilisation selon la revendication 6, **caractérisée en ce que** le matériau contient Ti et est utilisé comme catalyseur dans un procédé choisi parmi des procédés d'oxydation sélective de composés organiques en utilisant un agent oxydant choisi parmi H₂O₂ ou des peroxydes, des hydroperoxydes ou des peracides organiques.

38. Utilisation selon la revendication 6, **caractérisée en ce que** le matériau contient Sn et est utilisé comme catalyseur dans un procédé choisi parmi des procédés d'oxydation de type Baeyer-Villiger.

39. Utilisation selon la revendication 6, **caractérisée en ce que** le matériau est utilisé comme catalyseur dans un procédé choisi parmi des procédés d'oxydation de type Meerwein-Pondorf-Verley.

40. Utilisation selon la revendication 6, **caractérisée en ce que** le matériau est utilisé comme catalyseur dans un procédé choisi parmi des procèdes d'hydroisomérisation d'oléfines, d'alcoylation d'oléfines avec des isoparaffines et d'alcoylation d'aromatiques avec des oléfines ou des alcools.
